# EUROPEAN PATENT SPECIFICATION

(11) **EP 2 611 442 B1**
(45) Date of publication and mention of the grant of the patent: **04.07.2018**
(21) Application number: 11758019.1
(22) Date of filing: 01.09.2011
(51) Int. Cl.: A61K 31/403, A61K 31/155, A61K 9/32, A61K 9/36, A61K 9/34, A61K 47/12, A61K 9/20, A61K 9/28, A61K 47/02, A61K 47/10, A61K 47/14, A61K 45/06, A61K 9/24, A61K 31/4985, A61K 31/70

(54) **DRUG FORMULATIONS USING WATER SOLUBLE ANTIOXIDANTS**
WIRKSTOFFFORMULIERUNGEN MIT WASERLÖSLICHEN ANTIOXIDANZIEN
FORMULATIONS DE MÉDICAMENT AU MOYEN D'ANTIOXYDANTS HYDROSOLUBLES

(30) Priority: 03.09.2010 US 379970 P
(43) Date of publication of application: 10.07.2013
(73) Proprietor: Bristol-Myers Squibb Company, Princeton, NJ 08543 (US); AstraZeneca UK Limited, London W1K 1LN (GB)
(72) Inventor: NARANG, Ajit, New Brunswick, NJ 08903 (US); RAO, Venkatramana, M., New Brunswick, NJ 08903 (US); DESAI, Divyakant, S., New Brunswick, NJ 08903 (US)
(74) Representative: Reitstötter Kinzebach
(86) International application number: PCT/US2011/050212
(87) International publication number: WO 2012/031124

(56) References cited:
- WO-A1-02/102376
- WO-A1-2009/099734
- WO-A1-2009/111200
- US-A1- 2010 074 950
- MIKHAL'CHUK V.M., KRYUK T.V., PETRENKO L.V., NELEPOVA O.A., NIKOLAEVSKII A.N.: "Antioxidative Stabilization of Polyethylene Glycol in Aqueous Solutions with Herb Phenols", RUSSIAN JOURNAL OF APPLIED CHEMISTRY, vol. 77, no. 1, 2004, pages 131-135, XP002674221, Russia

## Description

### FIELD OF THE INVENTION

The present invention relates to solid formulations comprising water soluble antioxidants that prevent or reduce formic acid and/or formyl species generation in the dosage form during the manufacturing process and/or during shelf-life storage. The formulations of the present invention prevent or reduce formation of N-formyl impurities (and gelatin crosslinking) during the manufacturing process and/or during shelf-life storage.

### BACKGROUND OF THE INVENTION

Saxagliptin is a dipeptidyl peptidase IV (DPP-IV) inhibitor currently being used for the treatment of type 2 diabetes and is marketed under the trade name Onglyza®.
Saxagliptin tablets, 2.5 mg and 5 mg, have been developed using an active coating process. In this process, an inert tablet core is film-coated with three layers of pH-adjusted Opadry® II-based coating suspension. The first layer forms the seal-coat and contains Opadry white; the second layer contains saxagliptin and Opadry white; and the third, outer, layer contains Opadry with color, to distinguish different strengths.

In addition, fixed dose combination (FDC) tablets of saxagliptin with metformin hydrochloride immediate release (Met IR) and extended release (Met XR) tablets have been developed using the same active coating process by replacing inert tablets cores with the Met IR or Met XR core tablets. The FDC tablets are intended to improve patient compliance by providing two drugs with different modes of action in a single tablet.

In tablet form, saxagliptin degrades via intra-molecular cyclization to form a cyclic amidine (CA) as described in WO 2005/117841. Formulating saxagliptin with Opadry® II white as the second coating reduces the formation of CA. However, during the process of preparing the second coating, an N-formyl adduct (NFA) impurity is observed. The primary amine of saxagliptin is formylated by the reaction of saxagliptin with a formylating species such as formic acid, formic acid esters or formates, and/or formic acid anhydrides. Therefore, a need exists to prevent or reduce the degradation of saxagliptin during the manufacturing process as well as during shelf-life storage.
Pharmaceutical compositions comprising a DPP-IV inhibitor are described in US 2010/0074950 A1, WO 2009/099734 A1 and WO 2009/111200 A1.

The present invention provides formulations comprising water soluble antioxidants that prevent or reduce primary and/or secondary amino groups of active pharmaceutical ingredients from forming NFAs. In particular, the formulations of the present invention prevent or reduce formylation of saxagliptin during the manufacturing process and/or during shelf-life storage.

### SUMMARY OF THE INVENTION

The present invention provides solid formulations comprising at least one water soluble antioxidant. The formulations of the present invention prevent or reduce formation of formaldehyde, formic acid, and/or derivates thereof during the manufacturing process and/or during shelf-life storage thereby preventing or reducing formyl impurities such as NFAs. Specifically, the present invention provides a coated tablet comprising:
(a) a tablet core wherein the tablet core comprises
   (i) optionally at least one antidiabetic agent or a pharmaceutically acceptable salt thereof, wherein the antidiabetic agent is other than saxagliptin;
(b) a first layer that coats the tablet core, wherein the first layer comprises
   (i) a coating material; and
   (ii) optionally at least one water soluble antioxidant;
(c) a second layer that coats the first layer wherein the second layer comprises
   (i) a coating material comprising at least about 25 wt% of a combination of poly(ethylene glycol) and poly(vinyl alcohol);
   (ii) at least one water soluble antioxidant selected from ascorbic acid, propyl gallate, or a combination thereof; and
   (iii) saxagliptin or a pharmaceutically acceptable salt thereof; and
(d) a third layer that coats the second layer wherein the third layer comprises
   (i) a coating material; and
   (ii) optionally at least one water soluble antioxidant.
Further, solid and semisolid formulations are described herein which include, but are not limited to, tablet, stock granulation, or capsule formulations. Further, liquid formulations are described herein which include, but are not limited to, coating formulations, during the manufacturing process, that have a propensity to form or support the formation of formaldehyde, formic acid, and/or derivatives thereof.

The present disclosure provides tablet formulations that contain a tablet core coated with three layers. The tablet core may be inert or may contain one or more antidiabetic agent(s). The first layer coats the tablet core and comprises a coating material and optionally at least one water soluble antioxidant. The second layer coats the first layer and comprises an active pharmaceutical ingredient, that is a primary amine or a secondary amine, in combination with a coating material and at least one water soluble antioxidant. The third layer coats the second layer and comprises a coating material and optionally at least one water soluble antioxidant.

Further, the present disclosure provides immediate release and extended release tablet formulations. The tablet core comprises an antidiabetic agent and three coatings. The first layer coats the tablet core and comprises a coating material and optionally at least one water soluble antioxidant. The second layer coats the first layer and comprises an active pharmaceutical ingredient, that is a primary amine or a secondary amine, in combination with a coating material and at least one water soluble antioxidant. The third layer coats the second layer and comprises a coating material and optionally at least one water soluble antioxidant.

The present disclosure also provides methods of preparing solid, semisolid, or liquid formulations that prevent or reduce N-formylation of an active pharmaceutical ingredient that is a primary amine or a secondary amine comprising adding at least one water soluble antioxidant to the formulation.

The present disclosure also provides a method of preventing or reducing the formation of formic acid in solid, semisolid, or liquid formulations comprising adding at least one water soluble antioxidant to the formulation.

### BRIEF DESCRIPTION OF THE DRAWINGS

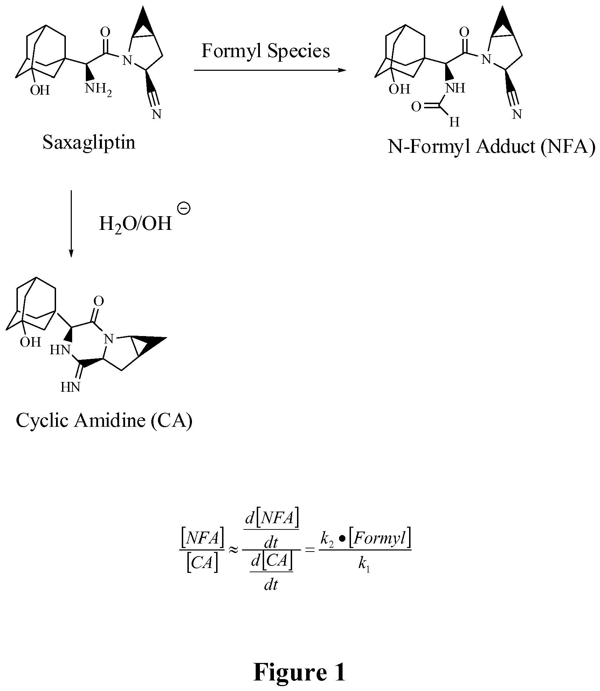

**Figure 1** illustrates the degradation products of saxagliptin.
**Figure 2** illustrates the possible degradation pathways of polyethylene glycol (PEG) to provide formylating species.

### DETAILED DESCRIPTION OF THE DISCLOSURE

The present invention discloses the use of water-soluble antioxidant(s) in solid formulations to prevent or reduce N-formylation of the amine containing active pharmaceutical ingredient saxagliptin. Amine drugs, such as saxagliptin, can react with formic acid or its derivatives to form an NFA impurity. The water soluble antioxidants may react with formic acid or its derivatives to prevent or reduce N-formylation. Alternatively, the antioxidants may inhibit oxidative degradation of polyethylene glycol (PEG) and/or other excipients in the formulation that lead to the formation of formic acid.

It has been surprisingly found that oxidative generation of formic acid impacts long-term drug product stability following short-term exposure of drug product to high temperature/humidity conditions or storage of the coating dispersion (which contains PEG) during drug product manufacture. Formic acid formation is thought to follow a free radical mechanism and it is known that butylated hydroxyanisole (BHA) or butylated hydroxytoluene (BHT) prevents or reduces free radical-mediated oxidation reactions. Further, it has been unexpectedly found that water-soluble antioxidants, such as ascorbic acid or propyl gallate, are more effective in preventing or reducing the formation of formic acid than antioxidants such as BHA and BHT which are poorly soluble in water.

The present invention also includes the use of one or more water-soluble antioxidant(s) in combination with one or more low water soluble antioxidant(s) to ensure drug product stability. Water-soluble antioxidants prevent or reduce formic acid generation during drug product manufacture and during exposure to high temperature/humidity conditions. Poorly water soluble antioxidants provide long-term drug product stability in the solid state resulting in longer shelf-life for these drug products.

Factors that may contribute to N-formylation of active pharmaceutical ingredients include temperature, humidity, amount of formylating species present, and the microenvironment effects in solid-state reactivity. In the liquid state, a bimolecular reaction involving an amine containing active pharmaceutical ingredient and formic acid (or a formate or formaldehyde) to form the NFA is directly proportional to the concentrations of the reacting species. In the solid-state, temperature and moisture play a role by increasing the plasticity and molecular mobility of the reacting species. The origin of the formylating species may be attributed to degradation of the excipients during the process of manufacturing the tablet (or liquid formulation) or during storage.

Formyl impurities in drug products may be controlled by preventing or reducing the presence of the formylating species in the formulations. However, there are significant challenges to controlling the level of formylating species generated during the manufacturing process and/or during shelf-life storage. For example, the type and relative proportion of formylating species are unknown. Among the species that may be present are formic acid, formates, acetic formic anhydride, and possibly other unknown species. These different species are expected to have different reactivity in a bimolecular reaction with an amine drug.

Development of analytical methods for the quantization of formyl species is challenging. The formyl species detection methods usually require derivatization with an alcohol, such as ethanol, to form an ester followed by high performance liquid chromatography (HPLC) or gas chromatography (GC) separation and detection. These methods are non-specific with respect to type and relative proportion of formyl species present in the starting materials.

The formylating species may be present in the excipients before the formulation process begins. For example, polyvinyl alcohol (PVA) used in the coatings of the present invention is manufactured from polyvinyl acetate by hydrolysis. The PVA polymer that is commercially available and used for film coating process contains residual acetic acid. In addition, relatively high levels of formic acid can be present in the incoming PVA as an impurity. The presence of both acetic acid and formic acid in the coating formulation may facilitate formation of the highly reactive acetic formic anhydride.

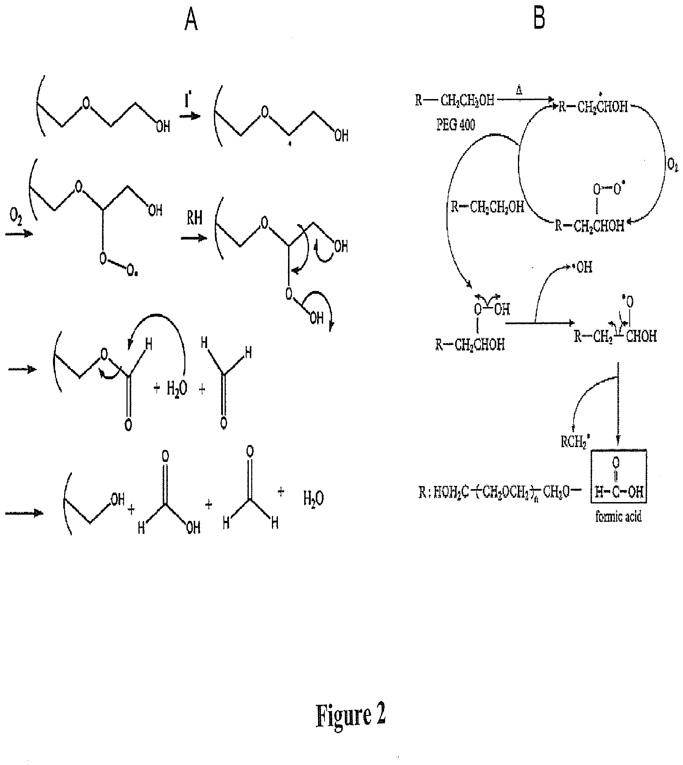

Formic acid is produced in polyethylene glycol (PEG) upon exposure to high temperatures and humidity conditions and is thought to be the result of an oxidative mechanism involving free radicals (Figure 2). In addition, levels of formic acid increase on storage when PEG and PVA are combined, as compared to PEG alone.

In capsule dosage forms or capsule formulations, the formaldehyde generated from PEG reacts with gelatin to cross link the gelatin in addtion to N-formylating the active ingredient. Crosslinked gelatin shells do not dissolve in aqueous medium easily leading to slowdown or lack of capsule shell dissolution and release of the active ingredient. Cross linking of gelatin is encountered in hard gelatin capsule dosage form development with solid formulation ingredients that include higher (>2000) molecular weights of PEG that are solid at room temperature. Cross linking is also encountered in liquid or semisolid filled soft gelatin formulations that comprise lower (<2000) molecular weights of PEGs that are liquid or semisolid at room temperature. The use of water soluble antioxidants in capsule shell formulations and/or drug product formulations prevent or reduce cross linking and/or N-formylation of the active ingredient.

Antioxidants can be categorized into three general categories based on their mechanism of action. There are initiation inhibitors that prevent the initiation phase of the free radical-mediated chain reaction and/or remove catalytic initiators. These are exemplified by the chelating agents, such as ethylenediamine tetraacetic acid (EDTA). There are free radical terminators that react with free radicals and inhibit the propagation phase of the free radical chain reaction. These are exemplified by BHA and BHT. Finally, there are reducing agents that possess lower redox potential than the oxidation substrate in the formulation leading to their preferential oxidization. These are exemplified by ascorbic acid, thiols (such as thioglycerol and thioglycollic acid), and polyphenols (such as propyl gallate).

Long term shelf-life stability in the solid-state is usually the basis of selection of antioxidants for use in oral solid dosage forms, such as tablets, and in commercial coating materials, such as Opadry. For this purpose, the use of free radical chain reaction terminators, such as BHT, are preferred. Reports suggest that this would also be the case for preventing oxidative generation of formic acid in tablet formulations with active pharmaceutical ingredients susceptible to N-formylation such as saxagliptin tablets.

It has been found that the formylation species can increase in tablet formulations during three different phases: shelf-life storage of Opadry (coating material) as a powder (PEG in the solid-state); shelf-life storage of the coated tablet (PEG in the solid-state); and manufacture of the tablet formulations (PEG in the solution state), known as the suspension hold time. In addition, it has been found that N-formylation increases in tablets after an initial, short-term exposure of the drug to high temperature/humidity conditions for a short period of time (Example 1).

The initial, short term oxidation promoting conditions that can cause initiation of a self-propagating chain reaction include the hold time storage of the Opadry suspension. As shown in Example 2, formyl species can increase relatively rapidly in Opadry suspension stored at room temperature. This initial increase in the formation of formyl species in the Opadry suspension can have implications on the drug product stability in finished packages. As shown in Example 3, the storage of Opadry suspension for up to 72 hours at room temperature during routine production operation could lead to higher NFA formation in the finished drug product.

Although BHT has been shown to effectively prevent formic acid growth in PEG in the solid-state, it may not be as effective in preventing formic acid generation in the solution state and in plasticized Opadry films, which can have higher molecular mobility of reactive species compared to the dry powder. In these cases, the use of a water soluble antioxidant, such as ascorbic acid or propyl gallate, can offer significant advantages (Example 4).

The examples described herein show the tendency for formic acid generation during the storage of Opadry® II suspension and the long term storage effects of initial, short term exposure to higher temperature and humidity conditions. The greater effectiveness of water soluble antioxidants in preventing formic acid generation in Opadry films is also shown. Effective inhibition of the oxidation process during the drug product manufacturing or initial excursion can be critical to the long term, shelf-life storage stability of the drug product.

Accordingly, pharmaceutical dosage forms are described herein including a primary or secondary amine-containing active pharmaceutical ingredient and a water soluble antioxidant in substantial admixture. That is, the primary or secondary amine-containing active pharmaceutical ingredient and water soluble antioxidant are in intimate contact with one another, such that the chemical environment of each is substantially identical. Preferably, the primary or secondary amine-containing active pharmaceutical ingredient and the water soluble antioxidant are homogenously mixed. Of course, there can in certain cases be some degree of inhomogeneity; for example, particles of the pharmaceutical ingredient and/or particles of the water soluble antioxidant can be part of a substantially admixed material. The primary or secondary amine-containing active pharmaceutical ingredient and the water soluble antioxidant can be dispensed together, for example, in a tablet core, in a particulate or granulate formulation or in an active coating layer. The water soluble antioxidant can be provided at a concentration in the range of, for example, about 0.001 wt% to about 1 wt%, or about 0.01 to about 0.5 wt% of the material in which it is dispensed.

In certain cases, the primary or secondary amine-containing active pharmaceutical ingredient and the water soluble antioxidant are in substantial admixture with a poly(ethylene glycol), a poly(vinyl alcohol), or a combination thereof. For example, in certain cases, the amine compound and the primary or secondary amine-containing active pharmaceutical ingredient are part of substantially admixed material that is at least about 10%, at least about 25%, or even at least about 50% poly(ethylene glycol), poly(vinyl alcohol), or a combination thereof (calculated on a wt/wt basis, excluding solvent). In certain cases, the primary or secondary amine-containing active pharmaceutical ingredient and the water soluble antioxidant are in substantial admixture with a poly(ethylene glycol), a poly(vinyl alcohol), or both, as is the case in the active coating layers based on Opadry® II material as described below. Accordingly, in the formulations described herein, the coating material in which the primary or secondary amine-containing active pharmaceutical ingredient is dispensed (e.g., the coating material of the second layer) may comprise at least about 10 wt%, at least about 25 wt%, or even at least about 50 wt% poly(ethylene glycol), poly(vinyl alcohol), or a combination thereof.

A variety of water soluble antioxidants can be used. According to the invention, the water soluble antioxidant is ascorbic acid, propyl gallate, or a combination thereof. Further water soluble antioxidants described herein include sodium sulfite, sodium metabisulfite, sodium bisulfite, thioglycerol, thioglycollic acid, and a combination thereof. In certain embodiments, the water soluble antioxidant is propyl gallate. In other embodiments, the water soluble antioxidant is ascorbic acid. As the person of skill in the art will appreciate, the water soluble antioxidant can be provided as a combination of water soluble antioxidants.

According to the ivention, the amine-containing active pharmaceutical ingredient is saxagliptin. Further primary or secondary amine-containing active pharmaceutical ingredients described herein include sitagliptin, vildagliptin, linagliptin, dutogliptin, or alogliptin. The active pharmaceutical ingredient can be provided as its free base, as a pharmaceutically acceptable salt, or as a combination thereof. For example, the active pharmaceutical ingredient can be substantially provided as an ammonium salt or as a hydrochloride salt.

Certain formulations of the disclosure as described above are described in further detail below with respect to a variety of dosage forms.

Further, the present disclosure provides a method of preventing or reducing N-formylation of active pharmaceutical ingredients in a substantially admixed solid, semisolid, or liquid formulation material comprising adding a water-soluble antioxidant to the formulation material. The solid, semisolid, or liquid formulation materials can comprise a poly(ethylene glycol), a poly(vinyl alcohol), or a combination thereof. For example, the formulations can include poly(ethylene glycol), optionally in combination with poly(vinyl alcohol), as is the case in the Opadry® II material. The active pharmaceutical ingredient can be a primary or secondary amine. Preferred active pharmaceutical ingredients are selected from saxagliptin, sitagliptin, vildagliptin, linagliptin, dutogliptin, and alogliptin. The active pharmaceutical ingredient used according to the present invention is saxagliptin. Preferred water-soluble antioxidants include those described above. The water-soluble antioxidants used according to the present invention are propyl gallate and ascorbic acid. The teachings of the present disclosure as described with respect to dosage forms can be applied to the methods of the present invention by the person of skill in the art.

### INERT TABLET CORE FORMULATIONS (TABLE 12)

The present disclosure provides tablet formulations that comprise a tablet core that optionally comprises at least one antidiabetic or a pharmaceutically acceptable salt thereof, where the antidiabetic is other than saxagliptin. A first layer coats the tablet core and comprises a coating material and optionally at least one water soluble antioxidant. A second layer coats the first layer and comprises an active pharmaceutical ingredient, a coating material, and at least one water soluble antioxidant, where the active pharmaceutical ingredient is a primary or secondary amine. A third layer coats the second layer and comprises a coating material and optionally a water soluble antioxidant.

The present disclosure also provides tablet formulations that comprise a tablet core that optionally comprises at least one antidiabetic or a pharmaceutically acceptable salt thereof, where the antidiabetic is other than saxagliptin. A first layer coats the tablet core and comprises a coating material and optionally at least one water soluble antioxidant. A second layer coats the first layer and comprises an active pharmaceutical ingredient, a coating material, and at least one water soluble antioxidant, where the active pharmaceutical ingredient is saxagliptin, sitagliptin, vildagliptin, linagliptin, dutogliptin, or alogliptin. A third layer coats the second layer and comprises a coating material and optionally a water soluble antioxidant.

The present disclosure also provides tablet formulations that comprise a tablet core that optionally comprises at least one antidiabetic or a pharmaceutically acceptable salt thereof, where the antidiabetic is other than saxagliptin. A first layer coats the tablet core and comprises a coating material and optionally at least one water soluble antioxidant. A second layer coats the first layer and comprises an active pharmaceutical ingredient, a coating material, and at least one water soluble antioxidant, where the active pharmaceutical ingredient is saxagliptin. A third layer coats the second layer and comprises a coating material and optionally a water soluble antioxidant.

The present disclosure also provides tablet formulations that comprise a tablet core that optionally comprises at least one antidiabetic or a pharmaceutically acceptable salt thereof, where the antidiabetic is other than saxagliptin. A first layer coats the tablet core and comprises Opadry® II and optionally at least one water soluble antioxidant. A second layer coats the first layer and comprises an active pharmaceutical ingredient, Opadry® II, and at least one water soluble antioxidant, where the active pharmaceutical ingredient is saxagliptin. A third layer coats the second layer and comprises Opadry® II and optionally a water soluble antioxidant.

The present disclosure also provides tablet formulations that comprise a tablet core that optionally comprises at least one antidiabetic or a pharmaceutically acceptable salt thereof, where the antidiabetic is other than saxagliptin. A first layer coats the tablet core and comprises a coating material and optionally at least one water soluble antioxidant. A second layer coats the first layer and comprises an active pharmaceutical ingredient, a coating material, and at least one water soluble antioxidant, where the active pharmaceutical ingredient is saxagliptin. A third layer coats the second layer and comprises a coating material and optionally a water soluble antioxidant. The water soluble antioxidant is ascorbic acid, propyl gallate, sodium sulfite, sodium metabisulfite, sodium bisulfite, thioglycerol, thioglycollic acid, or a combination thereof.

The present disclosure also provides tablet formulations that comprise a tablet core that optionally comprises at least one antidiabetic or a pharmaceutically acceptable salt thereof, where the antidiabetic is other than saxagliptin. A first layer coats the tablet core and comprises Opadry® II and optionally at least one water soluble antioxidant. A second layer coats the first layer and comprises an active pharmaceutical ingredient, Opadry® II, and at least one water soluble antioxidant, where the active pharmaceutical ingredient is saxagliptin. A third layer coats the second layer and comprises Opadry® II and optionally a water soluble antioxidant. The water soluble antioxidant is ascorbic acid, propyl gallate, sodium sulfite, sodium metabisulfite, sodium bisulfite, thioglycerol, thioglycollic acid, or a combination thereof.

In one aspect, the present invention provides tablet formulations that comprise a tablet core that optionally comprises at least one antidiabetic or a pharmaceutically acceptable salt thereof, where the antidiabetic is other than saxagliptin. A first layer coats the tablet core and comprises a coating material and optionally at least one water soluble antioxidant. A second layer coats the first layer and comprises an active pharmaceutical ingredient, a coating material comprising at least about 25 wt% of a combination of poly(ethylene glycol) and poly(vinyl alcohol), and at least one water soluble antioxidant, where the active pharmaceutical ingredient is saxagliptin. A third layer coats the second layer and comprises a coating material and optionally a water soluble antioxidant. The water soluble antioxidant is ascorbic acid or propyl gallate.

In another aspect, the present invention provides tablet formulations that comprise a tablet core that optionally comprises at least one antidiabetic or a pharmaceutically acceptable salt thereof, where the antidiabetic is other than saxagliptin. A first layer coats the tablet core and comprises Opadry® II and optionally at least one water soluble antioxidant. A second layer coats the first layer and comprises an active pharmaceutical ingredient, Opadry® II, and at least one water soluble antioxidant, where the active pharmaceutical ingredient is saxagliptin. A third layer coats the second layer and comprises Opadry® II and optionally a water soluble antioxidant. The water soluble antioxidant is ascorbic acid or propyl gallate.

In another aspect, the present invention provides tablet formulations that comprise a tablet core that comprises two fillers, a disintegrant, and a lubricant. A first layer coats the tablet core and comprises Opadry® II and optionally at least one water soluble antioxidant. A second layer coats the first layer and comprises an active pharmaceutical ingredient, Opadry® II, and at least one water soluble antioxidant, where the active pharmaceutical ingredient is saxagliptin. A third layer coats the second layer and comprises Opadry® II and optionally a water soluble antioxidant. The water soluble antioxidant is ascorbic acid or propyl gallate.

In another aspect, the present invention provides tablet formulations that comprise a tablet core that comprises two fillers, a disintegrant, and a lubricant. A first layer coats the tablet core and comprises Opadry® II. A second layer coats the first layer and comprises an active pharmaceutical ingredient, Opadry® II, and at least one water soluble antioxidant, where the active pharmaceutical ingredient is saxagliptin and the water soluble antioxidant is ascorbic acid or propyl gallate. A third layer coats the second layer and comprises Opadry® II.

The present disclosure also provides tablet formulations that comprise a tablet core that comprises two fillers, a disintegrant, and a lubricant. A first layer coats the tablet core and comprises Opadry® II. A second layer coats the first layer and comprises an active pharmaceutical ingredient, Opadry® II, and at least one water soluble antioxidant, where the active pharmaceutical ingredient is sitagliptin and the water soluble antioxidant is ascorbic acid or propyl gallate. A third layer coats the second layer and comprises Opadry® II.

The present disclosure also provides tablet formulations that comprise a tablet core that comprises two fillers, a disintegrant, and a lubricant. A first layer coats the tablet core and comprises Opadry® II. A second layer coats the first layer and comprises an active pharmaceutical ingredient, Opadry® II, and at least one water soluble antioxidant, where the active pharmaceutical ingredient is vildagliptin and the water soluble antioxidant is ascorbic acid or propyl gallate. A third layer coats the second layer and comprises Opadry® II.

In another aspect, the present invention provides tablet formulations that comprise a tablet core that comprises lactose monohydrate, microcrystalline cellulose, croscarmellose sodium, and magnesium stearate. A first layer coats the tablet core and comprises Opadry® II white. A second layer coats the first layer and comprises saxagliptin, Opadry® II white, and propyl gallate. A third layer coats the second layer and comprises Opadry® II color.

The present disclosure also provides tablet formulations that comprise a tablet core that comprises lactose monohydrate, microcrystalline cellulose, croscarmellose sodium, and magnesium stearate. A first layer coats the tablet core and comprises Opadry® II white. A second layer coats the first layer and comprises sitagliptin, Opadry® II white, and propyl gallate. A third layer coats the second layer and comprises Opadry® II color.

The present disclosure also provides tablet formulations that comprise a tablet core that comprises lactose monohydrate, microcrystalline cellulose, croscarmellose sodium, and magnesium stearate. A first layer coats the tablet core and comprises Opadry® II white. A second layer coats the first layer and comprises vildagliptin, Opadry® II white, and propyl gallate. A third layer coats the second layer and comprises Opadry® II color.

### IMMEDIATE RELEASE FORMULATIONS (TABLE 13)

The present disclosure provides immediate release tablet formulations that comprise a tablet core that comprises a binder, a wetting agent, a lubricant, and optionally at least one antidiabetic or a pharmaceutically acceptable salt thereof, where the antidiabetic is other than saxagliptin. A first layer coats the tablet core and comprises a coating material and optionally at least one water soluble antioxidant. A second layer coats the first layer and comprises an active pharmaceutical ingredient, a coating material, and at least one water soluble antioxidant, where the active pharmaceutical ingredient is a primary or secondary amine. A third layer coats the second layer and comprises a coating material and optionally a water soluble antioxidant.

The present disclosure also provides immediate release tablet formulations that comprise a tablet core that comprises a binder, a wetting agent, a lubricant, and at least one antidiabetic or a pharmaceutically acceptable salt thereof, where the antidiabetic is an alpha glucosidase inhibitors, insulin, a meglitinide, a sulfonylurea, a biguanide, a biguanide/glyburide combination, a thiazolidinedione, a PPAR-alpha agonist, a PPAR-gamma agonist, a PPAR alpha/gamma dual agonist, a glycogen phosphorylase inhibitor, an inhibitor of fatty acid binding protein (aP2), a GPR-119 modulators, a GPR 40 modulator, a glucokinase inhibitor, a glucagon-like peptide-1 (GLP-1) and other agonists of the GLP-1 receptor, a SGLT2 inhibitor, dipeptidyl peptidase IV (DPP4) inhibitor other than saxagliptin, or combinations thereof. A first layer coats the tablet core and comprises a coating material and optionally at least one water soluble antioxidant. A second layer coats the first layer and comprises an active pharmaceutical ingredient, a coating material, and at least one water soluble antioxidant, where the active pharmaceutical ingredient is a primary or secondary amine. A third layer coats the second layer and comprises a coating material and optionally a water soluble antioxidant.

The present disclosure also provides immediate release tablet formulations that comprise a tablet core that comprises a binder, a wetting agent, a lubricant, and at least one antidiabetic or a pharmaceutically acceptable salt thereof, where the antidiabetic is acarbose, miglitol, insulin, repaglinide, nateglinide, KAD1229, acetohexamide, chlorpropamide, glibenclamide (glyburide), gliclazide, glimepiride, glipizide, glyclopyramide, tolazamide, tolbutamide, buformin, metformin, phenformin, Glucovance®, rosiglitazone, pioglitazone, troglitazone, MCC-555, faraglitazar, englitazone, darglitazone, CP-86325, isaglitazone, reglitazar, JTT-501, rivoglitazone, R-119702, liraglutide, (Z)-1,4-bis-4-[(3,5-dioxo-1,2,4-oxadiazolidin-2-yl-methyl)]phenoxybut-2-ene, YM-440, , muraglitazar, peliglitazar, tesaglitazar AR-HO39242, GW-501516, KRP297, sitagliptin, vildagliptin, linagliptin, dutogliptin, and alogliptin, NVP-DPP728A (1-[[[2-[(5-cyanopyridin-2-yl)amino]ethyl]amino]acetyl]-2-cyano-(S)-pyrrolidine), TSL-225 (tryptophyl-1,2,3,4-tetrahydroisoquinoline-3-carboxylic acid, canagliflozin, dapagliflozin, dapagliflozin (S) propylene glycol hydrate, remogliflozin, sergliflozin or combinations thereof. A first layer coats the tablet core and comprises a coating material and optionally at least one water soluble antioxidant. A second layer coats the first layer and comprises an active pharmaceutical ingredient, a coating material, and at least one water soluble antioxidant, where the active pharmaceutical ingredient is a primary or secondary amine. A third layer coats the second layer and comprises a coating material and optionally a water soluble antioxidant.

The present disclosure also provides immediate release tablet formulations that comprise a tablet core that comprises a binder, a wetting agent, a lubricant, and at least one antidiabetic or a pharmaceutically acceptable salt thereof, where the antidiabetic is glibenclamide (glyburide), metformin, Glucovance®, rosiglitazone, pioglitazone, sitagliptin, vildagliptin, dapagliflozin, dapagliflozin (S) propylene glycol hydrate, or combinations thereof. A first layer coats the tablet core and comprises a coating material and optionally at least one water soluble antioxidant. A second layer coats the first layer and comprises an active pharmaceutical ingredient, a coating material, and at least one water soluble antioxidant, where the active pharmaceutical ingredient is a primary or secondary amine. A third layer coats the second layer and comprises a coating material and optionally a water soluble antioxidant.

The present disclosure also provides immediate release tablet formulations that comprise a tablet core that comprises a binder, a wetting agent, a lubricant, and at least one antidiabetic or a pharmaceutically acceptable salt thereof, where the antidiabetic is glyburide, metformin, Glucovance®, rosiglitazone, pioglitazone, sitagliptin, vildagliptin, dapagliflozin, dapagliflozin (S) propylene glycol hydrate, or combinations thereof. A first layer coats the tablet core and comprises a coating material and optionally at least one water soluble antioxidant. A second layer coats the first layer and comprises an active pharmaceutical ingredient, a coating material, and at least one water soluble antioxidant, where the active pharmaceutical ingredient is saxagliptin, sitagliptin, vildagliptin, linagliptin, dutogliptin, or alogliptin. A third layer coats the second layer and comprises a coating material and optionally a water soluble antioxidant.

The present disclosure also provides immediate release tablet formulations that comprise a tablet core that comprises a binder, a wetting agent, a lubricant, and at least one antidiabetic or a pharmaceutically acceptable salt thereof, where the antidiabetic is glyburide, metformin, Glucovance®, rosiglitazone, pioglitazone, sitagliptin, vildagliptin, dapagliflozin, dapagliflozin (S) propylene glycol hydrate, or combinations thereof. A first layer coats the tablet core and comprises Opadry® II and optionally at least one water soluble antioxidant. A second layer coats the first layer and comprises an active pharmaceutical ingredient, Opadry® II, and at least one water soluble antioxidant, where the active pharmaceutical ingredient is saxagliptin, sitagliptin, vildagliptin, linagliptin, dutogliptin, or alogliptin. A third layer coats the second layer and comprises Opadry® II and optionally a water soluble antioxidant.

The present disclosure also provides immediate release tablet formulations that comprise a tablet core that comprises a binder, a wetting agent, a lubricant, and at least one antidiabetic or a pharmaceutically acceptable salt thereof, where the antidiabetic is metformin, dapagliflozin, dapagliflozin (S) propylene glycol hydrate, or combinations thereof. A first layer coats the tablet core and comprises a coating material and optionally at least one water soluble antioxidant. A second layer coats the first layer and comprises an active pharmaceutical ingredient, a coating material, and at least one water soluble antioxidant, where the active pharmaceutical ingredient is saxagliptin. A third layer coats the second layer and comprises a coating material and optionally a water soluble antioxidant.

The present disclosure also provides immediate release tablet formulations that comprise a tablet core that comprises a binder, a wetting agent, a lubricant, and at least one antidiabetic or a pharmaceutically acceptable salt thereof, where the antidiabetic is metformin, dapagliflozin, dapagliflozin (S) propylene glycol hydrate, or combinations thereof. A first layer coats the tablet core and comprises Opadry® II and optionally at least one water soluble antioxidant. A second layer coats the first layer and comprises an active pharmaceutical ingredient, Opadry® II, and at least one water soluble antioxidant, where the active pharmaceutical ingredient is saxagliptin. A third layer coats the second layer and comprises Opadry® II and optionally a water soluble antioxidant.

The present disclosure also provides immediate release tablet formulations that comprise a tablet core that comprises a binder, a wetting agent, a lubricant, and at least one antidiabetic or a pharmaceutically acceptable salt thereof, where the antidiabetic is metformin, dapagliflozin, dapagliflozin (S) propylene glycol hydrate, or combinations thereof. A first layer coats the tablet core and comprises a coating material and optionally at least one water soluble antioxidant. A second layer coats the first layer and comprises an active pharmaceutical ingredient, a coating material, and at least one water soluble antioxidant, where the active pharmaceutical ingredient is saxagliptin. A third layer coats the second layer and comprises a coating material and optionally a water soluble antioxidant. The water soluble antioxidant is ascorbic acid, propyl gallate, sodium sulfite, sodium metabisulfite, sodium bisulfite, thioglycerol, thioglycollic acid, or a combination thereof.

The present disclosure also provides immediate release tablet formulations that comprise a tablet core that comprises a binder, a wetting agent, a lubricant, and at least one antidiabetic or a pharmaceutically acceptable salt thereof, where the antidiabetic is metformin, dapagliflozin, dapagliflozin (S) propylene glycol hydrate, or combinations thereof. A first layer coats the tablet core and comprises Opadry® II and optionally at least one water soluble antioxidant. A second layer coats the first layer and comprises an active pharmaceutical ingredient, Opadry® II, and at least one water soluble antioxidant, where the active pharmaceutical ingredient is saxagliptin. A third layer coats the second layer and comprises Opadry® II and optionally a water soluble antioxidant. The water soluble antioxidant is ascorbic acid, propyl gallate, sodium sulfite, sodium metabisulfite, sodium bisulfite, thioglycerol, thioglycollic acid, or a combination thereof.

In one aspect, the present invention provides immediate release tablet formulations that comprise a tablet core that comprises a binder, a wetting agent, a lubricant, and at least one antidiabetic or a pharmaceutically acceptable salt thereof, where the antidiabetic is metformin, dapagliflozin, dapagliflozin (S) propylene glycol hydrate, or combinations thereof. A first layer coats the tablet core and comprises Opadry® II and optionally at least one water soluble antioxidant. A second layer coats the first layer and comprises an active pharmaceutical ingredient, Opadry® II, and at least one water soluble antioxidant, where the active pharmaceutical ingredient is saxagliptin. A third layer coats the second layer and comprises Opadry® II and optionally a water soluble antioxidant. The water soluble antioxidant is ascorbic acid or propyl gallate.

In another aspect, the present invention provides immediate release tablet formulations that comprise a tablet core that comprises a binder, a wetting agent, a lubricant, and dapagliflozin or dapagliflozin (S) propylene glycol hydrate. A first layer coats the tablet core and comprises Opadry® II. A second layer coats the first layer and comprises saxagliptin, Opadry® II, and ascorbic acid or propyl gallate. A third layer coats the second layer and comprises Opadry® II.

In another aspect, the present invention provides immediate release tablet formulations that comprise a tablet core that comprises povidone, purified water, magnesium stearate, and dapagliflozin or dapagliflozin (S) propylene glycol hydrate. A first layer coats the tablet core and comprises Opadry® II white. A second layer coats the first layer and comprises saxagliptin, Opadry® II white, and ascorbic acid or propyl gallate. A third layer coats the second layer and comprises Opadry® II color.

In another aspect, the present invention provides immediate release tablet formulations that comprise a tablet core that comprises a binder, a wetting agent, a lubricant, and metformin hydrochloride. A first layer coats the tablet core and comprises Opadry® II white. A second layer coats the first layer and comprises saxagliptin, Opadry® II white, and ascorbic acid or propyl gallate. A third layer coats the second layer and comprises Opadry® II color.

In another aspect, the present invention provides immediate release tablet formulations that comprise a tablet core that comprises povidone, purified water, magnesium stearate, and metformin hydrochloride. A first layer coats the tablet core and comprises Opadry® II white. A second layer coats the first layer and comprises saxagliptin, Opadry® II white, and ascorbic acid or propyl gallate. A third layer coats the second layer and comprises Opadry® II color.

### EXTENDED RELEASE FORMULATIONS (TABLE 14)

The present disclosure provides extended release tablet formulations that comprise a tablet core that comprises a disintegrant, a release modifier, a filler, a wetting agent, a lubricant, and optionally at least one antidiabetic or a pharmaceutically acceptable salt thereof, where the antidiabetic is other than saxagliptin. A first layer coats the tablet core and comprises a coating material and optionally at least one water soluble antioxidant. A second layer coats the first layer and comprises an active pharmaceutical ingredient, a coating material, and at least one water soluble antioxidant, where the active pharmaceutical ingredient is a primary or secondary amine. A third layer coats the second layer and comprises a coating material and optionally a water soluble antioxidant.

The present disclosure also provides extended release tablet formulations that comprise a tablet core that comprises a disintegrant, a release modifier, a filler, a wetting agent, a lubricant, and at least one antidiabetic or a pharmaceutically acceptable salt thereof, where the antidiabetic is an alpha glucosidase inhibitors, insulin, a meglitinide, a sulfonylurea, a biguanide, a biguanide/glyburide combination, a thiazolidinedione, a PPAR-alpha agonist, a PPAR-gamma agonist, a PPAR alpha/gamma dual agonist, a glycogen phosphorylase inhibitor, an inhibitor of fatty acid binding protein (aP2), a GPR-119 modulators, a GPR 40 modulator, a glucokinase inhibitor, a glucagon-like peptide-1 (GLP-1) and other agonists of the GLP-1 receptor, a SGLT2 inhibitor, dipeptidyl peptidase IV (DPP4) inhibitor other than saxagliptin, or combinations thereof. A first layer coats the tablet core and comprises a coating material and optionally at least one water soluble antioxidant. A second layer coats the first layer and comprises an active pharmaceutical ingredient, a coating material, and at least one water soluble antioxidant, where the active pharmaceutical ingredient is a primary or secondary amine. A third layer coats the second layer and comprises a coating material and optionally a water soluble antioxidant.

The present disclosure also provides extended release tablet formulations that comprise a tablet core that comprises a disintegrant, a release modifier, a filler, a wetting agent, a lubricant, and at least one antidiabetic or a pharmaceutically acceptable salt thereof, where the antidiabetic is acarbose, miglitol, insulin, repaglinide, nateglinide, KAD1229, acetohexamide, chlorpropamide, glibenclamide (glyburide), gliclazide, glimepiride, glipizide, glyclopyramide, tolazamide, tolbutamide, buformin, metformin, phenformin, Glucovance®, rosiglitazone, pioglitazone, troglitazone, MCC-555, faraglitazar, englitazone, darglitazone, CP-86325, isaglitazone, reglitazar, JTT-501, rivoglitazone, R-119702, liraglutide, (Z)-1,4-bis-4-[(3,5-dioxo-1,2,4-oxadiazolidin-2-yl-methyl)]phenoxybut-2-ene, YM-440,, muraglitazar, peliglitazar, tesaglitazar AR-HO39242, GW-501516, KRP297, sitagliptin, vildagliptin, linagliptin, dutogliptin, and alogliptin, NVP-DPP728A (1-[[[2-[(5-cyanopyridin-2-yl)amino]ethyl]amino]acetyl]-2-cyano-(S)-pyrrolidine), TSL-225 (tryptophyl-1,2,3,4-tetrahydroisoquinoline-3-carboxylic acid, canagliflozin, dapagliflozin, dapagliflozin (S) propylene glycol hydrate, remogliflozin, sergliflozin or combinations thereof. A first layer coats the tablet core and comprises a coating material and optionally at least one water soluble antioxidant. A second layer coats the first layer and comprises an active pharmaceutical ingredient, a coating material, and at least one water soluble antioxidant, where the active pharmaceutical ingredient is a primary or secondary amine. A third layer coats the second layer and comprises a coating material and optionally a water soluble antioxidant.

The present disclosure also provides extended release tablet formulations that comprise a tablet core that comprises a disintegrant, a release modifier, a filler, a wetting agent, a lubricant, and at least one antidiabetic or a pharmaceutically acceptable salt thereof, where the antidiabetic is glibenclamide (glyburide), metformin, Glucovance®, rosiglitazone, pioglitazone, sitagliptin, vildagliptin, dapagliflozin, dapagliflozin (S) propylene glycol hydrate, or combinations thereof. A first layer coats the tablet core and comprises a coating material and optionally at least one water soluble antioxidant. A second layer coats the first layer and comprises an active pharmaceutical ingredient, a coating material, and at least one water soluble antioxidant, where the active pharmaceutical ingredient is a primary or secondary amine. A third layer coats the second layer and comprises a coating material and optionally a water soluble antioxidant.

The present disclosure also provides extended release tablet formulations that comprise a tablet core that comprises a disintegrant, a release modifier, a filler, a wetting agent, a lubricant, and at least one antidiabetic or a pharmaceutically acceptable salt thereof, where the antidiabetic is glyburide, metformin, Glucovance®, rosiglitazone, pioglitazone, sitagliptin, vildagliptin, dapagliflozin, dapagliflozin (S) propylene glycol hydrate, or combinations thereof. A first layer coats the tablet core and comprises a coating material and optionally at least one water soluble antioxidant. A second layer coats the first layer and comprises an active pharmaceutical ingredient, a coating material, and at least one water soluble antioxidant, where the active pharmaceutical ingredient is saxagliptin, sitagliptin, vildagliptin, linagliptin, dutogliptin, or alogliptin. A third layer coats the second layer and comprises a coating material and optionally a water soluble antioxidant.

The present disclosure also provides extended release tablet formulations that comprise a tablet core that comprises a disintegrant, a release modifier, a filler, a wetting agent, a lubricant, and at least one antidiabetic or a pharmaceutically acceptable salt thereof, where the antidiabetic is glyburide, metformin, Glucovance®, rosiglitazone, pioglitazone, sitagliptin, vildagliptin, dapagliflozin, dapagliflozin (S) propylene glycol hydrate, or combinations thereof. A first layer coats the tablet core and comprises Opadry® II and optionally at least one water soluble antioxidant. A second layer coats the first layer and comprises an active pharmaceutical ingredient, Opadry® II, and at least one water soluble antioxidant, where the active pharmaceutical ingredient is saxagliptin, sitagliptin, vildagliptin, linagliptin, dutogliptin, or alogliptin. A third layer coats the second layer and comprises Opadry® II and optionally a water soluble antioxidant.

The present disclosure also provides extended release tablet formulations that comprise a tablet core that comprises a disintegrant, a release modifier, a filler, a wetting agent, a lubricant, and at least one antidiabetic or a pharmaceutically acceptable salt thereof, where the antidiabetic is metformin, dapagliflozin, dapagliflozin (S) propylene glycol hydrate, or combinations thereof. A first layer coats the tablet core and comprises a coating material and optionally at least one water soluble antioxidant. A second layer coats the first layer and comprises an active pharmaceutical ingredient, a coating material, and at least one water soluble antioxidant, where the active pharmaceutical ingredient is saxagliptin. A third layer coats the second layer and comprises a coating material and optionally a water soluble antioxidant.

The present disclosure also provides extended release tablet formulations that comprise a tablet core that comprises a disintegrant, a release modifier, a filler, a wetting agent, a lubricant, and at least one antidiabetic or a pharmaceutically acceptable salt thereof, where the antidiabetic is metformin, dapagliflozin, dapagliflozin (S) propylene glycol hydrate, or combinations thereof. A first layer coats the tablet core and comprises Opadry® II and optionally at least one water soluble antioxidant. A second layer coats the first layer and comprises an active pharmaceutical ingredient, Opadry® II, and at least one water soluble antioxidant, where the active pharmaceutical ingredient is saxagliptin. A third layer coats the second layer and comprises Opadry® II and optionally a water soluble antioxidant.

The present disclosure also provides extended release tablet formulations that comprise a tablet core that comprises a disintegrant, a release modifier, a filler, a wetting agent, a lubricant, and at least one antidiabetic or a pharmaceutically acceptable salt thereof, where the antidiabetic is metformin, dapagliflozin, dapagliflozin (S) propylene glycol hydrate, or combinations thereof. A first layer coats the tablet core and comprises a coating material and optionally at least one water soluble antioxidant. A second layer coats the first layer and comprises an active pharmaceutical ingredient, a coating material, and at least one water soluble antioxidant, where the active pharmaceutical ingredient is saxagliptin. A third layer coats the second layer and comprises a coating material and optionally a water soluble antioxidant. The water soluble antioxidant is ascorbic acid, propyl gallate, sodium sulfite, sodium metabisulfite, sodium bisulfite, thioglycerol, thioglycollic acid, or a combination thereof.

The present disclosure also provides extended release tablet formulations that comprise a tablet core that comprises a disintegrant, a release modifier, a filler, a wetting agent, a lubricant, and at least one antidiabetic or a pharmaceutically acceptable salt thereof, where the antidiabetic is metformin, dapagliflozin, dapagliflozin (S) propylene glycol hydrate, or combinations thereof. A first layer coats the tablet core and comprises Opadry® II and optionally at least one water soluble antioxidant. A second layer coats the first layer and comprises an active pharmaceutical ingredient, Opadry® II, and at least one water soluble antioxidant, where the active pharmaceutical ingredient is saxagliptin. A third layer coats the second layer and comprises Opadry® II and optionally a water soluble antioxidant. The water soluble antioxidant is ascorbic acid, propyl gallate, sodium sulfite, sodium metabisulfite, sodium bisulfite, thioglycerol, thioglycollic acid, or a combination thereof.

In one aspect, the present invention provides extended release tablet formulations that comprise a tablet core that comprises a disintegrant, a release modifier, a filler, a wetting agent, a lubricant, and at least one antidiabetic or a pharmaceutically acceptable salt thereof, where the antidiabetic is metformin, dapagliflozin, dapagliflozin (S) propylene glycol hydrate, or combinations thereof. A first layer coats the tablet core and comprises Opadry® II and optionally at least one water soluble antioxidant. A second layer coats the first layer and comprises an active pharmaceutical ingredient, Opadry® II, and at least one water soluble antioxidant, where the active pharmaceutical ingredient is saxagliptin. A third layer coats the second layer and comprises Opadry® II and optionally a water soluble antioxidant. The water soluble antioxidant is ascorbic acid or propyl gallate.

In another aspect, the present invention provides extended release tablet formulations that comprise a tablet core that comprises a disintegrant, a release modifier, a filler, a wetting agent, a lubricant, and dapagliflozin or dapagliflozin (S) propylene glycol hydrate. A first layer coats the tablet core and comprises Opadry® II white. A second layer coats the first layer and comprises saxagliptin, Opadry® II white, and ascorbic acid or propyl gallate. A third layer coats the second layer and comprises Opadry® II color.

In another aspect, the present invention provides extended release tablet formulations that comprise a tablet core that comprises sodium carboxymethylcellulose, hydroxypropyl methylcellulose, microcrystalline cellulose, purified water, magnesium stearate, and dapagliflozin or dapagliflozin (S) propylene glycol hydrate. A first layer coats the tablet core and comprises Opadry® II white. A second layer coats the first layer and comprises saxagliptin, Opadry® II white, and ascorbic acid or propyl gallate. A third layer coats the second layer and comprises Opadry® II color.

In another aspect, the present invention provides extended release tablet formulations that comprise a tablet core that comprises a disintegrant, a release modifier, a filler, a wetting agent, a lubricant, and metformin hydrochloride. A first layer coats the tablet core and comprises Opadry® II white. A second layer coats the first layer and comprises saxagliptin, Opadry® II white, and ascorbic acid or propyl gallate. A third layer coats the second layer and comprises Opadry® II color.

In another aspect, the present invention provides extended release tablet formulations that comprise a tablet core that comprises sodium carboxymethylcellulose, hydroxypropyl methylcellulose, microcrystalline cellulose, purified water, magnesium stearate, and metformin hydrochloride. A first layer coats the tablet core and comprises Opadry® II white. A second layer coats the first layer and comprises saxagliptin, Opadry® II white, and ascorbic acid or propyl gallate. A third layer coats the second layer and comprises Opadry® II color.

The present disclosure also provides a method of preventing or reducing N-formylation of active pharmaceutical ingredients in solid, semisolid, or liquid formulations comprising adding at least one water soluble antioxidant to the formulation. The solid, semisolid, or liquid formulations comprise polyethylene glycol alone or in combination with polyvinyl alcohol such as with Opadry® II. The active pharmaceutical ingredient is a primary or secondary amine. Preferred active pharmaceutical ingredients are selected from saxagliptin, sitagliptin, vildagliptin, linagliptin, dutogliptin, and alogliptin. The most preferred active pharmaceutical ingredient is saxagliptin. Preferred water soluble antioxidants are selected from ascorbic acid, propyl gallate, sodium sulfite, sodium metabisulfite, sodium bisulfite, thioglycerol, thioglycollic acid, and combinations thereof. The most preferred water soluble antioxidant is selected from ascorbic acid and propyl gallate. The present disclosure also contemplates water soluble antioxidants in combination with poorly water soluble antioxidants or antioxidants that are less than 0.1 mg/mL soluble in water. Poorly water soluble antioxidants include, but are not limited to, butylated hydroxyanisole, butylated hydroxytoluene, alpha-tocopherol, beta-tocopherol, gama-tocopherol, delta-tocopherol, and ascorbyl palmitate.

The present disclosure also provides a method of preventing or reducing the generation of formic acid in solid, semisolid, or liquid formulations comprising adding at least one water soluble antioxidant to the formulation. The solid, semisolid, or liquid formulations comprise polyethylene glycol alone or in combination with polyvinyl alcohol including, but not limited to, Opadry® II. The active pharmaceutical ingredient is a primary or secondary amine. Preferred active pharmaceutical ingredients are selected from saxagliptin, sitagliptin, vildagliptin, linagliptin, dutogliptin, and alogliptin. The most preferred active pharmaceutical ingredient is saxagliptin. Preferred water soluble antioxidants are selected from ascorbic acid, propyl gallate, sodium sulfite, sodium metabisulfite, sodium bisulfite, thioglycerol, thioglycollic acid, and combinations thereof. The most preferred water soluble antioxidant is selected from ascorbic acid and propyl gallate. The present invention also contemplates water soluble antioxidants in combination with poorly water soluble antioxidants or antioxidants that are less than 0.1 mg/mL soluble in water. Poorly water soluble antioxidants include, but are not limited to, butylated hydroxyanisole, butylated hydroxytoluene, alpha-tocopherol, beta-tocopherol, gama-tocopherol, delta-tocopherol, and ascorbyl palmitate.

The present disclosure also contemplates the use of a modified release coating comprising a coating material, at least one modified release component, optionally an active pharmaceutical ingredient, and optionally a water soluble antioxidant. The time release characteristics for the release of the active ingredient(s) from each or either of the regions (such as tablet core or coating) may be varied by modifying the composition of each component, including modifying any of the excipients or coatings which may be present. In particular the release of the active ingredient may be controlled by changing the composition and/or the amount of the modified release coating on the particles, if such a coating is present. If more than one modified release component is present, the modified release coating for each of these components may be the same or different. Similarly, when modified release is facilitated by the inclusion of a modified release matrix material, release of the active ingredient may be controlled by the choice and amount of modified release matrix material utilised. The modified release coating may be present, in each component, in any amount that is sufficient to yield the desired delay time for each particular component. The modified release coating may be preset, in each component, in any amount that is sufficient to yield the desired time lag between components. The lag time or delay time for the release of the active ingredient from each component may also be varied by modifying the composition of each of the components, including modifying any excipients and coatings which may be present. For example the first component may be an immediate release component wherein the active ingredient is released substantially immediately upon administration. Alternatively, the first component may be, for example, a time-delayed immediate release component in which the active ingredient is released substantially immediately after a time delay. The second component may be, for example, a time-delayed immediate release component as just described or, alternatively, a time-delayed sustained release or extended release component in which the active ingredient is released in a controlled fashion over an extended period of time.

Binders suitable for use in the formulations of the present invention include, but are not limited to, methyl cellulose, carboxymethyl cellulose (including sodium carboxymethyl cellulose), hydroxypropyl cellulose (including HPC-SSL, HPC-SL, HPC-L, HPC-EXF, etc.), hydroxypropylmethyl cellulose, corn starch, pregelatinized starch, modified corn starch, polyvinyl pyrrolidone (PVP), hydroxypropyl methylcellulose (HPMC) (including hydroxypropyl methylcellulose 2208), lactose, gum acacia, gum arabic, gelatin, agar, ethyl cellulose, cellulose acetate, tragacanth, sodium alginate, pullulan, as well as a wax binder such as carnauba wax, paraffin, spermaceti, polyethylenes or microcrystalline wax, as well as other conventional binding agents and/or mixtures of two or more thereof. Preferred binders of the present invention are hydroxypropyl cellulose, hydroxypropyl methylcellulose, starch, and polyvinyl pyrrolidone.

Disintegrants suitable for use in the formulations of the present invention include, but are not limited to, croscarmellose sodium, crospovidone, starch, potato starch, pregelatinized starch, corn starch, sodium starch glycolate, microcrystalline cellulose, low substituted hydroxypropyl cellulose LH21, polyvinyl pyrrolidone cross linked, and other known disintegrants. Several specific types of disintegrant are suitable for use in the formulations described herein. For example, any grade of crospovidone can be used, including for example crospovidone XL-10, and includes members selected from the group consisting of Kollidon CL®, Polyplasdone XL®, Kollidon CL-M®, Polyplasdone XL-10®, and Polyplasdone INF-10®. In one embodiment, the disintegrant, if present, of the stock granulation is sodium starch glycolate, croscarmellose sodium and/or crospovidone. The preferred disintegrants are croscarmellose sodium, crospovidone, starch, and sodium starch glycolate.

Fillers suitable for use in the formulations of the present invention include, but are not limited to, cellulose derivatives, such as microcrystalline cellulose or wood cellulose (including microcrystalline cellulose 302), lactose, lactose anhydrous, sucrose, starch, pregelatinized starch, dextrose, mannitol (including mannitol Pearlitol SD 200), fructose, xylitol, sorbitol, corn starch, modified corn starch, inorganic salts such as calcium carbonate, calcium phosphate, dicalcium phosphate, calcium sulfate, dextrin/dextrates, maltodextrin, compressible sugars, and other known bulking agents or fillers, and/or mixtures of two or more thereof. Several types of microcrystalline cellulose are suitable for use in the formulations described herein, for example, microcrystalline cellulose selected from the group consisting of Avicel® types: PH101, PH102, PH103, PH105, PH 112, PH113, PH200, PH301, and other types of microcrystalline cellulose, such as silicified microcrystalline cellulose. Several types of lactose are suitable for use in the formulations described herein, for example, lactose selected from the group consisting of anhydrous lactose, lactose monohydrate, lactose fast flow, directly compressible anhydrous lactose, and modified lactose monohydrate. The preferred fillers of the present invention are microcrystalline cellulose and lactose monohydrate.

Glidants and/or anti-adherents suitable for use in the formulations of the present invention include, but are not limited to, silicon dioxide, colloidal silicon dioxide, magnesium silicate, magnesium trisilicate, talc, and other forms of silicon dioxide, such as aggregated silicates and hydrated silica. Preferred glidants are talc, lecithin, and colloidal silicon dioxide.

Lubricants suitable for use in the formulations of the present invention include, but are not limited to, magnesium stearate, zinc stearate, calcium stearate, talc, carnauba wax, stearic acid, palmitic acid, sodium stearyl fumarate sodium laurel sulfate, glyceryl palmitostearate, palmitic acid, myristic acid and hydrogenated vegetable oils and fats, as well as other known lubricants, and/or mixtures of two or more thereof. The preferred lubricants of the present invention are magnesium stearate and stearic acid.

Release modifiers suitable for use in the formulations of the present invention include, but are not limited to, sodium alginate, hydroxypropyl methylcellulose (HPMC), hydroxypropyl cellulose (HPC), methyl cellulose (MC), ethyl cellulose (EC), acrylate polymers, any grades of Eudragit® such as Eudragit RL or RS, poly acrylic acid and poly acrylate and methacrylate copolymers such as those sold under the Trade Mark Eudragite S and L, polyvinyl acetaldiethylamino acetate, hydroxypropyl methylcellulose acetate succinate, shellac; hydrogels and gel-forming materials, such as carboxyvinyl polymers, sodium alginate, sodium carmellose, calcium carmellose, sodium carboxymethyl starch, poly vinyl alcohol, hydroxyethyl cellulose, methyl cellulose, gelatin, starch, and cellulose based cross-linked polymers-in which the degree of crosslinking is low so as to facilitate adsorption of water and expansion of the polymer matrix, hydoxypropyl cellulose, hydroxypropyl methylcellulose, polyvinylpyrrolidone, crosslinked starch, microcrystalline cellulose, chitin, aminoacryl-methacrylate copolymer (Eudragit® RS-PM, Rohm & Haas), pullulan, collagen, casein, agar, gum arabic, sodium carboxymethyl cellulose, (swellable hydrophilic polymers) poly(hydroxyalkyl methacrylate) (m. wt. ^{∼}5 k-5,000 k), polyvinylpyrrolidone (m. wt. ^{∼}10 k-360 k), anionic and cationic hydrogels, polyvinyl alcohol having a low acetate residual, a swellable mixture of agar and carboxymethyl cellulose, copolymers of maleic anhydride and styrene, ethylene, propylene or isobutylene, pectin (m. wt. ^{∼}30 k-300 k), polysaccharides such as agar, acacia, karaya, tragacanth, algins and guar, polyacrylamides, Polyox® polyethylene oxides (m. wt. ^{∼}100 k-5,000 k), AquaKeep® acrylate polymers, diesters of polyglucan, crosslinked polyvinyl alcohol and poly N-vinyl-2-pyrrolidone, sodium starch glucolate (e.g. Explotab®; Edward Mandell C. Ltd.); hydrophilic polymers such as polysaccharides, methyl cellulose, sodium or calcium carboxymethyl cellulose, hydroxypropyl methyl cellulose, hydroxypropyl cellulose, hydroxyethyl cellulose, nitro cellulose, carboxymethyl cellulose, cellulose ethers, polyethylene oxides (e.g. Polyox®, Union Carbide), methyl ethyl cellulose, ethylhydroxy ethylcellulose, cellulose acetate, cellulose butyrate, cellulose propionate, gelatin, collagen, starch, maltodextrin, pullulan, polyvinyl pyrrolidone, polyvinyl alcohol, polyvinyl acetate, glycerol fatty acid esters, polyacrylamide, polyacrylic acid, copolymers of methacrylic acid or methacrylic acid (e.g. Eudragit®, Rohm and Haas), other acrylic acid derivatives, sorbitan esters, natural gums, lecithins, pectin, alginates, ammonia alginate, sodium, calcium, potassium alginates, propylene glycol alginate, agar, and gums such as arabic, karaya, locust bean, tragacanth, carrageens, guar, xanthan, scleroglucan and mixtures and blends thereof. Wetting agents suitable for use in the formulations of the present invention include, but are not limited to, polysorbate, polyvinylpyrrolidone, anionic surfactants (based on permanent anions such as sulfate, sulfonate, phosphate or pH-dependent anions such as carboxylate) such as ammonium lauryl sulfate, sodium lauryl sulfate (SDS), sodium laureth sulfate (also known as sodium lauryl ether sulfate (SLES)), sodium myreth sulfate; dioctyl sodium sulfosuccinate, perfluorooctanesulfonate (PFOS), perfluorobutanesulfonate; alkyl benzene sulfonates; alkyl aryl ether phosphates, alkyl ether phosphates, alkyl carboxylates such as fatty acid salts (soaps), sodium stearate, sodium lauroyl sarcosinate; carboxylate fluorosurfactants: perfluorononanoate, perfluorooctanoate; cationic surfactants that are pH-dependent (e.g., ,primary, secondary or tertiary amines) such as octenidine dihydrochloride; permanently charged quaternary ammonium cation such as alkyltrimethylammonium salts, e.g., cetyl trimethylammonium bromide (CTAB) or hexadecyl trimethyl ammonium bromide, cetyl trimethylammonium chloride (CTAC); cetylpyridinium chloride (CPC); polyethoxylated tallow amine (POEA); benzalkonium chloride (BAC); benzethonium chloride (BZT); 5-Bromo-5-nitro-1,3-dioxane; dimethyldioctadecylammonium chloride; dioctadecyldimethylammonium bromide (DODAB); zwitterionic or amphoteric surfactants such as sulfonates, e.g., CHAPS (3-[(3-Cholamidopropyl)dimethylammonio] -1-propanesulfonate), sultaines: cocamidopropyl hydroxysultaine; carboxylates, such as amino acids, imino acids, betaines, e.g., cocamidopropyl betaine; phosphates such as lecithin; nonionic surfactants such as fatty alcohols, e.g., cetyl alcohol, stearyl alcohol, cetostearyl alcohol (consisting predominantly of cetyl and stearyl alcohols), oleyl alcohol; polyoxyethylene glycol alkyl ethers (Brij): CH₃-(CH₂)₁₀₋₁₆-(O-C₂H₄)₁₋₂₅-OH: octaethylene glycol monododecyl ether, pentaethylene glycol monododecyl ether; polyoxypropylene glycol alkyl ethers: CH₃-(CH₂)₁₀₋₁₆-(O-C₃H₆)₁₋₂₅-OH; glucoside alkyl ethers: CH₃-(CH₂)₁₀₋₁₆-(O-Glucoside)₁₋₃-OH: decyl glucoside, lauryl glucoside, octyl glucoside; polyoxyethylene glycol octylphenol ethers: C₈H₁₇-(C₆H₄)-(O-C₂H₄)₁₋₂₅-OH: triton X-100; polyoxyethylene glycol alkylphenol ethers: C₉H₁₉-(C₆H₄)-(O-C₂H₄)₁₋₂₅-OH: oonoxynol-9; glycerol alkyl esters such as glyceryl laurate; polyoxyethylene glycol sorbitan alkyl esters: polysorbates; sorbitan alkyl esters: spans; cocamide MEA, cocamide DEA; dodecyl dimethylamine oxide; and block copolymers of polyethylene glycol and polypropylene glycol, such as poloxamers.

The term "coating material" as used herein means any coating material that has the propensity to form, or support the formation of, formylating species including, but not limited to, formic acid, formates, formaldehyde, and/or derivatives thereof.

The term "Opadry® II color" includes, but is not limited to, Opadry® HP, Opadry® II white, Opadry® II orange, Opadry® II brown, or Opadry® II yellow. In addtion to Opadry II color, the third layer can also optionally include an anti-adherent or glidant such as talc, fumed silica, or magnesium stearate, or an opacifying agent, such as titanium dioxide. The third layer may optionally include combinations of one or more Opadry II colors. Preferred coatings of the present invention are Opadry® II white, Opadry® II brown, Opadry® II orange, and Opadry® II yellow.

The term "Opadry® HP " as used here means a film coating for a tablet that comprises 40% polyvinyl alcohol, 20% polyethylene glycol, 15% talc, and 25% titanium dioxide.

The term " Opadry® II" as used here means a film coating for a tablet that comprises polyvinyl alcohol, titanium dioxide, polyethylene glycol (PEG), and talc. Opadry® II white 85F18422 is comprised of polyvinyl alcohol, titanium dioxide, polyethylene glycol, and talc. Opadry® II Yellow 85F92582 is comprised of polyvinyl alcohol, titanium dioxide, polyethylene glycol, talc, and yellow iron dioxide.

The term "pharmaceutically acceptable salt" or "salt," as used herein, refers to salts that are well known in the art. For example, S. M Berge et al. describe pharmaceutically acceptable salts in detail in J. Pharmaceutical Sciences, 66:1-19 (1977). Examples of pharmaceutically acceptable salts include acetic acid, aspartic acid, benzenesulfonic acid, benzoic acid, butyric acid, citric acid, fumaric acid, hydrochloric acid, hydrobromic acid, lactic acid, maleic acid, malonic acid, methanesulfonic acid, 4-methylbenzenesulfonic acid, nicotinic acid, phosphoric acid, succinic acid, sulfuric acid, or tartaric acid, prepared by using methods well known in the art. The preferred pharmaceutically acceptable salt of saxagliptin for use in the methods of the present invention is HCl.

The term "poorly water soluble antioxidant" as used herein means any antioxidant with a water solubility of less than 0.1 mgs/mL.

The term "water soluble antioxidant" as used herein means any antioxidant with a water solubility equal to, or greater than, 0.1 mgs/mL.

Suitable anti-diabetic agents for use in the formulations of the present invention include, but are not limited to, alpha glucosidase inhibitors (acarbose or miglitol), insulins (including insulin secretagogues or insulin sensitizers), meglitinides (repaglinide, nateglinide, or KAD1229 (PF/Kissei)), sulfonylureas, biguanides (buformin, metformin, phenformin), biguanide/glyburide combinations (Glucovance®), thiazolidinediones, PPAR-alpha agonists, PPAR-gamma agonists, PPAR alpha/gamma dual agonists, glycogen phosphorylase inhibitors, inhibitors of fatty acid binding protein (aP2), GPR-119 modulators, GPR 40 modulators, glucokinase inhibitors, glucagon-like peptide-1 (GLP-1) and other agonists of the GLP-1 receptor, SGLT2 inhibitors, and dipeptidyl peptidase IV (DPP4) inhibitors other than saxagliptin. The antidiabetic agents used in the formulations of the present invention can be used in the amounts indicated in the Physician's Desk Reference or as in the cited patents and patent applications set out above or as otherwise known and used by one of ordinary skill in the art.

Suitable thiazolidinediones include, but are not limited to, rosiglitazone, pioglitazone, troglitazone, MCC-555 (disclosed in U.S. Patent No. 5,594,016, Mitsubishi), faraglitazar (GI-262570, Glaxo-Wellcome), englitazone (CP-68722, Pfizer) or darglitazone (CP-86325, Pfizer; isaglitazone, MIT/Johnson& Johnson), reglitazar (JTT-501, (JPNT/Pharmacia & Upjohn), rivoglitazone (R-119702, Sankyo/WL), liraglutide (NN-2344, Dr. Reddy/NN), and (Z)-1,4-bis-4-[(3,5-dioxo-1,2,4-oxadiazolidin-2-yl-methyl)]phenoxybut-2-ene (YM-440, Yamanouchi). The preferred thiazolidinediones are rosiglitazone and pioglitazone.

Suitable sulfonylureas include, but are not limited to, acetohexamide, chlorpropamide, glibenclamide (glyburide), gliclazide, glimepiride, glipizide, glyclopyramide, tolazamide, and tolbutamide. The preferred sulfonylurea is glyburide.

Suitable forms of the antidiabetic agent metformin for use in the present invention's formulations include pharmaceutically acceptable salts thereof such as the hydrochloride, hydrobromide, fumarate, succinate, p-chlorophenoxy acetate or embonate. The fumarate and succinate salts are preferably metformin (2:1) fumarate, and metformin (2:1) succinate. Metformin hydrochloride is the preferred salt.

Suitable PPAR-alpha agonists, PPAR-gamma agonists and PPAR alpha/gamma dual agonists include, but are not limited to, muraglitazar, peliglitazar, tesaglitazar AR-HO39242 (Astra/Zeneca), GW-501516 (Glaxo-Wellcome), KRP297 (Kyorin Merck), as well as those disclosed by Murakami et al, "A Novel Insulin Sensitizer Acts As a Coligand for Peroxisome Proliferation - Activated Receptor Alpha (PPAR alpha) and PPAR gamma. Effect on PPAR alpha Activation on Abnormal Lipid Metabolism in Liver of Zucker Fatty Rats", Diabetes 47, 1841-1847 (1998); WO 01/21602 and in U.S. Patent No. 6,414,002 and U.S Patent No. 6,653,314, employing dosages as set out therein. In one embodiment, the compounds designated as preferred in the cited references are preferred for use herein.

Suitable aP2 inhibitors include, but are not limited to, those disclosed in U.S. application Serial No. 09/391,053, filed September 7, 1999, and in U.S. Patent No. 6,548,529, employing dosages as set out therein.

Suitable DPP4 inhibitors include saxagliptin, sitagliptin, vildagliptin, linagliptin, dutogliptin, and alogliptin, as well as those disclosed in WO99/38501, WO99/46272, WO99/67279 (PROBIODRUG), WO99/67278 (PROBIODRUG), WO99/61431 (PROBIODRUG), NVP-DPP728A (1-[[[2-[(5-cyanopyridin-2-yl)amino]ethyl]amino]acetyl]-2-cyano-(S)-pyrrolidine) (Novartis) as disclosed by Hughes et al, Biochemistry, 38(36), 11597-11603, 1999, TSL-225 (tryptophyl-1,2,3,4-tetrahydroisoquinoline-3-carboxylic acid (disclosed by Yamada et al, Bioorg. & Med. Chem. Lett. 8 (1998) 1537-1540), 2-cyanopyrrolidides and 4-cyanopyrrolidides, as disclosed by Ashworth et al, Bioorg. & Med. Chem. Lett., Vol. 6, No. 22, pp 1163-1166 and 2745-2748 (1996), the compounds disclosed in U.S. application Serial No. 10/899,641, employing dosages as set out in the above references. Preferred DPP4 inhibitors for the tablet core (anti-diabetic agent) are selected from sitagliptin and vildagliptin. Saxagliptin is the preferred active pharmaceutical ingredient for the coatings or layers.

Saxagliptin or (1*S*,3*S*,5*S*)-2-[(2*S*)-2-amino-2-(3-hydroxyadamantan-1-yl)acetyl]-2-azabicyclo[3.1.0]hexane-3-carbonitrile can be prepared using the synthetic procedures described in US patent no. 6,395,767, in particular Example 60. The present invention encompasses pharmaceutically acceptable salts, as defined herein, of saxagliptin, in particular the benzoic acid, fumaric acid, hydrobromic acid, hydrochloric acid, methane sulfonic acid, tartaric acid, trifluoroacetic acid, salts of saxagliptin, for use in the methods described herein. Saxagliptin salts are prepared using techniques well known in the art or by using the procedures described in US patent nos. 6395767, and 7420079, and WO 08/131149. The methods of the present invention also encompass crystalline forms of saxagliptin as hydrates and/or solvates. In particular, the mono hydrate of saxagliptin is encompassed by the methods of the present invention. Other hydrate forms are also contemplated by the present invention including the hemi hydrate or (2:1) saxagliptin:H₂O. Hydrates of saxagliptin salts are also contemplated by the methods of the present invention and include the crystalline salt/hydrates disclosed in WO 08/131149. Saxagliptin inhibits DPP4, Ki=1.3 nM in Human DPP4 Inhibition.

Suitable SGLT2 inhibitors include canagliflozin, dapagliflozin, remogliflozin, and sergliflozin.

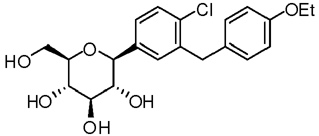

### Dapagliflozin

### (2S,3R,4R,5S,6R)-2-(4-chloro-3-(4-ethoxybenzyl)phenyl)-6-(hydroxymethyl)tetrahydro-2H-pyran-3,4,5-triol

Dapagliflozin can be prepared using similar procedures as described in U.S. Patent No. 6,515,117 or international published applications no. WO 03/099836 and WO 2008/116179. SGLT2 EC₅₀ = 1.1 nM.

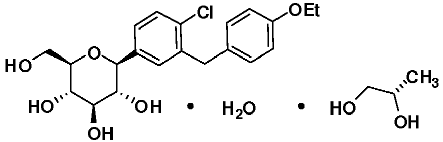

### Dapagliflozin (S) PGS

### (2S,3R,4R,5S,6R)-2-(4-chloro-3-(4-ethoxybenzyl)phenyl)-6-(hydroxymethyl)tetrahydro-2H-pyran-3,4,5-triol (S)-propane-1,2-diol hydrate (1:1:1)

Dapagliflozin (S) propylene glycol hydrate (1:1:1) can be prepared using similar procedures as described in published applications WO 08/002824 and WO 2008/116179. SGLT2 EC₅₀ = 1.1 nM.

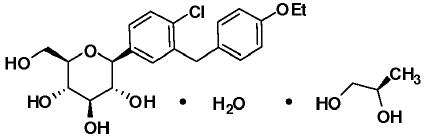

### Dapagliflozin (R) PGS

### (2S,3R,4R,5S,6R)-2-(4-chloro-3-(4-ethoxybenzyl)phenyl)-6-(hydroxymethyl)tetrahydro-2H-pyran-3,4,5-triol (R)-propane-1,2-diol hydrate (1:1:1)

Dapagliflozin (R) propylene glycol hydrate (1:1:1) can be prepared using similar procedures as described in WO 08/002824 and WO 2008/116179. SGLT2 EC₅₀ = 1.1 nM.

The present invention also contemplates formulations where the SGLT2 inhibitor is a compound of Formula (I) as described in U.S Patent No. 6,414,126. Other SGLT2 inhibitors contemplated by the present invention include sergliflozin, remogliflozin, etabonate, canagliflozin, BI-10773 and BI-44847, ASP-1941, R-7201, LX-4211, YM-543, AVE 2268, TS-033 or SGL-0100, and the compounds disclosed in US 7,589,193, WO2007007628, EP2009010, WO200903596, US2009030198, US 7,288,528 and US 2007/0197623. The following SGLT2 inhibitors, in addition to dapagliflozin, are preferred: 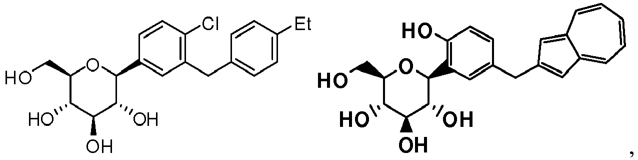 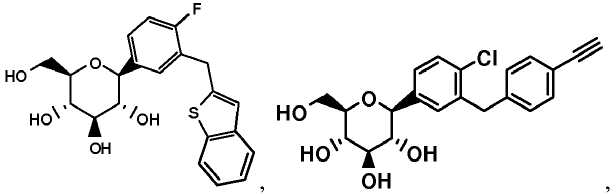 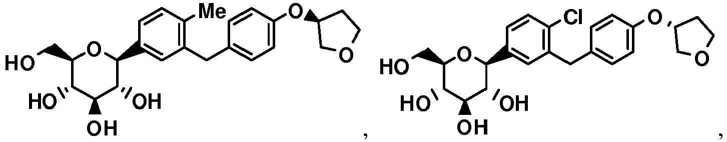 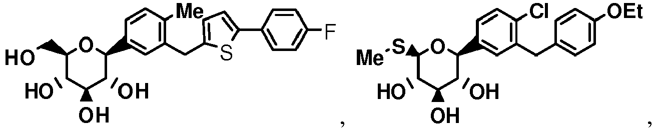 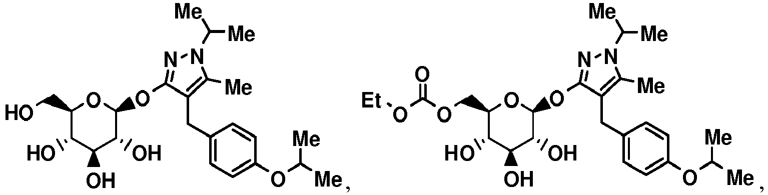 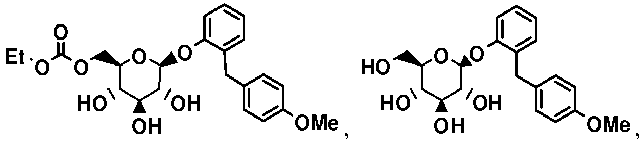 and

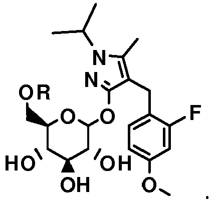

### Examples

### Example 1

Saxagliptin tablets 2.5 mg were exposed for one day to 60°C/75% RH and then stored at 30°C/65% RH for 12 months in HDPE bottles containing desiccant and induction sealed.

In a control study, saxagliptin tablets were stored in HDPE bottles at 30°C/65% RH for 12 months without prior exposure to 60°C/75% RH.

The NFA level in the exposed tablets was significantly higher than the unexposed tablets (∼ 0.4 - 0.5% w/w of saxagliptin versus < 0.05% w/w). The level of CA in both cases was similar (∼ 0.05% w/w of saxagliptin).

### Example 2

Four batches of Opadry® II white were used to prepare coating suspensions at 17.5% w/w concentration in water and adjusted to pH 2.0 ± 0.3 using hydrochloric acid, as per the manufacturing process of the saxagliptin second layer coating suspension. The suspensions were stored at room temperature (∼25°C) for 8 or 14 days. They were analyzed for total formyl species level by a derivatization gas chromatography (GC) method. The results are shown in Table 1.

**Table 1**

| **Formic Acid Level in Opadry Suspension Stored at Room Temperature** | | | |
|---|---|---|---|
| **Batch Number of Opadry® II White** | **Level of Formic Acid (ppm of Opadry Powder)** | | |
| | **Initial** | **Day 8** | **Day 14** |
| 1 | 112 | 427 | 582 |
| 2 | 130 | 627 | 960 |
| 3 | 50 | 225 | 339 |
| 4 | 132 | 305 | 446 |

The data in Table 1 shows a significant increase in formic acid levels in the suspensions of the coating material for all the Opadry® II white batches. Interpolation of this data to 72 hours of hold time storage, which is recommended by the vendor and usually followed in a drug product manufacturing operation, indicates a 1-2 fold increase in formic acid levels.

### Example 3

### Effect of Holding of Opadry Suspension on NFA Formation in Tablets.

Opadry® II white powder was dispersed in water at 17.5% w/w concentration in water and adjusted to pH 2.0 ± 0.3 using hydrochloric acid, as per the manufacturing process of saxagliptin second layer coating suspension. This Opadry suspension was used to prepare saxagliptin tablets 2.5 mg using the three layer coating procedure described earlier in Example 1, while holding the suspension for up to 72 hours (maximum hold time permitted during routine manufacturing) for each layer in one case and up to 12 hours (minimum possible during routine manufacturing) in another case. In the former case, the suspension was held for 48 hours before the initiation of coating, resulting in the total storage time of suspension (until the end of coating) of up to 72 hours. In the latter case, coating was initiated immediately after the preparation of suspension, resulting in the total storage time of suspension (until the end of coating) of up to 12 hours. The coated tablets were packaged in HDPE bottles with desiccants and induction sealed. The packaged drug product was kept at 40°C and 75% RH for 6 months or at 50°C for 1 month, followed by impurity analysis. The results of this study are shown in Table 2.

**Table 2**

| **Impurity Levels in Saxagliptin Tablets 2.5 mg with Holding of Opadry Suspension at Room Temperature for Different Periods of Time During Product Manufacturing.** | | | |
|---|---|---|---|
| **Impurity (% w/w of saxagliptin)** | **Storage condition in HDPE packs** | **Holding time of suspension (hours)** | |
| | | **12** | **72** |
| **% NFA** | **Initial** | < 0.05 | < 0.05 |
| | **40°C/75% RH/6 months** | 0.06 | **0.24** |
| | **50°C/1 month** | < 0.05 | **4.16** |
| **% CA** | **Initial** | < 0.05 | 0.08 |
| | **40°C/75% RH/6 months** | 0.09 | 0.19 |
| | **50°C/1 month** | < 0.05 | 0.26 |
| **Total** | **Initial** | 0.06 | 0.08 |
| | **40°C/75% RH/6 months** | 0.28 | 0.49 |
| | **50°C/1 month** | 0.07 | 5.31 |

After storage at 40°C/75% RH for 6 months, the NFA level in drug product that was stored for 72 hours at room temperature was found to be 0.24% w/w of saxagliptin, while it was 0.06% w/w for the drug product stored for 12 hours during product manufacturing. Similarly, after storage at 50°C for 1 month, the NFA level in drug product that was stored for 72 hours was found to be 4.16% w/w of saxagliptin, and <0.05% w/w for the drug product stored for 12 hours during product manufacturing.

In comparing the % CA and total impurites among batches with different hold times, NFA was found to be the major degradant that increased as a function of hold time of suspension. These data indicated that holding time of suspension during product manufacturing had an impact on the growth of NFA during drug product stability.

### Example 4

### Relative Effectiveness of Antioxidants in Reducing Formaldehyde and Formic acid Levels in Opadry Films

Opadry® II white powder was dispersed in water containing hydrochloric acid and adjusted to pH 2.0 ± 0.3. Five hundred µL of the coating suspension was dispensed in 5 mL scintillation vials. Open vials were kept overnight at 40°C to form a film. The vials were rubber stoppered and sealed. The sealed vials were kept at 60°C for 5 days and analyzed for formaldehyde and formic acid levels by a GC method.

In parallel experiments, Opadry suspension containing 0.2% w/v lipid soluble antioxidant (BHT) or 2% w/v water soluble antioxidant (ascorbic acid or propyl gallate) was prepared and treated similarly. These concentrations are on the higher end of the recommended use concentrations for each antioxidant and were used to test their maximum efficacy. The results of this study are shown in Table 3.

**Table 3**

| **Level of Formaldehyde and Formic Acid in Antioxidant Containing Opadry Films.** | | |
|---|---|---|
| **Antioxidant in the film** | **Level of formaldehyde (ppm)** | **Level of formic acid (ppm)** |
| None | 105 | 3848 |
| Butylated hydroxy toluene (BHT) | 95 | 1159 |
| Ascorbic acid | Not detected | 184 |
| Propyl gallate | Not detected | 60 |

The level of formaldehyde and formic acid in water soluble antioxidant containing samples was found to be significantly lower than in lipid soluble antioxidant containing samples. These results indicate significant inhibition of the oxidation process by water soluble antioxidants.

The water solubilities of BHA, BHT, ascorbic acid, and propyl gallate are shown below in Table 4.

**Table 4**

| **Antioxidant** | **Approximate water solubility at room temperature** | |
|---|---|---|
| | **mg/mL** | % w/v |
| Butylated hydroxy anisole (BHA) | 0.0151 | 0.00151 |
| Butylated hydroxy toluene (BHT) | 0.0006 - 0.0011 | 0.00006 - 0.00011 |
| Ascorbic acid | 330 | 33 |
| Propyl gallate | 3.33 | 0.33 |
| Sodium sulfite | 270 | 27 |

### Example 5

### Coating Formulation

A typical coating formulation for film coating of oral solid dosage forms, such as an immediate release tablet, is exemplified in Table 5.

**Table 5**

| **Typical Coating Formulation for Film Coating a Tablet** | | | |
|---|---|---|---|
| **Functional category of the component** | **Examples of components** | **Range (% w/w of total coating material)** | **Preferred Range (% w/w of total coating material)** |
| Polymer | Hydroxypropyl methylcellulose (HPMC), polyvinyl alcohol (PVA), ethyl cellulose (EC), methyl cellulose (MC), hydroxypropyl cellulose (HPC), acrylate polymers (e.g., Eudragits) | 25 - 55 | 35 - 45 |
| Plasticizer | Polyethylene glycol (PEG), glycerin, castor oil, propyl gallate (PG), surfactants | 1-50 | 20 - 30 |
| Opacifier | Titanium dioxide, pigment, aluminum lakes, iron oxides, natural colors | 0 - 40 | 10 - 30 |
| Glidant | Talc, lecithin | 0-15 | 5-15 |

Based on the findings of this invention, a proposed typical coating formulation for film coating of oral solid dosage forms, such as an immediate release tablet, containing an active pharmaceutical ingredient that is a primary or secondary amine susceptible to N-formylation is exemplified in Table 6.

**Table 6**

| **Proposed Typical Coating Composition for Film Coating a Tablet** | | | |
|---|---|---|---|
| **Functional Category of the Component** | **Components** | **Range (% w/w of total coating material)** | **Preferred Range (% w/w of total coating material)** |
| Polymer | Hydroxypropyl methylcellulose (HPMC), polyvinyl alcohol (PVA), ethyl cellulose (EC), methyl cellulose (MC), hydroxypropyl cellulose (HPC), acrylate polymers (e.g., Eudragits) | 25 - 55 | 35 - 45 |
| Plasticizer | Polyethylene glycol (PEG), glycerin, castor oil, propyl gallate (PG), surfactants | 1-50 | 20 - 30 |
| Opacifier | Titanium dioxide, pigment, aluminum lakes, iron oxides, natural colors | 0 - 40 | 10 - 30 |
| Glidant | Talc, lecithin | 0-15 | 5-15 |
| Polymer | Hydroxypropyl methylcellulose (HPMC), polyvinyl alcohol (PVA), ethyl cellulose (EC), methyl cellulose (MC), hydroxypropyl cellulose (HPC), acrylate polymers (e.g., Eudragits) | 25 - 55 | 35 - 45 |

### Reference Example 6

### Tablet Formulation with Drug in the Core

The findings disclosed in this invention are also applicable to oral solid dosage forms with drug in the core of the tablet. The formulations of this invention can be used for drugs that react with formic acid or a formylating species, such as formic acid esters or anhydrides. Water soluble antioxidants may be added to formulations that contain residual levels of formic acid or a different formylating species (in the range of 5 ppm or higher). The formulations may contain a formylating species initially and/or they may be formed on storage at high temperature (such as 40 °C to 60 °C) and/or humidity (such as 75% RH to 95% RH) conditions over a period of 2 days to 3 months. This tablet formulation may or may not be coated. If a tablet is coated, the antioxidant proposed in this invention can be used either in the coating or in the core of the tablet or both.

An example of such a coated immediate release tablet formulation is listed in Table 7. Based on the findings of this invention, a proposed formulation composition for the example cited in Table 7 would include a water soluble antioxidant, such as ascorbic acid, sodium ascorbate, propyl gallate, sodium sulfite, sodium bisulfite, sodium metabisulfite, thioglycerol, or thioglycollic acid. This antioxidant can be incorporated in the core of the tablet (Table 8), the coating material (Table 9) or both (Table 10). Further, more than one water soluble antioxidant can be used together. This may be done to reduce the amount of each individual antioxidant in the formulation.

The combination of a water soluble antioxidant and a water insoluble antioxidant can also be used. This may be done in cases where the antioxidants have different reactivities and/or stabilities. For example, incorporation of a water soluble antioxidant in the suspension formulation prevents an increase in any formylating species during suspension hold time and the use of water insoluble antioxidants in the coating material prevents an increase in the formylating species in the solid state. The ranges of concentration in which these antioxidants may be used in a drug product formulation are listed in Table 11.

**Table 7**

| **Typical Composition of an Immediate Release Tablet Formulation** | | | | |
|---|---|---|---|---|
| **Location in tablet** | **Functional class** | **Examples of components** | **Range*** | **Preferred Range*** |
| Core | Active pharmaceutical ingredient | Saxagliptin, sitagliptin, metformin | 1-98 | 5-65 |
| | Filler | Lactose monohydrate, microcrystalline cellulose | 5 - 95 | 20 - 80 |
| | Binder | Hydroxypropyl cellulose (HPC), polyvinyl pyrrolidone (PVP), starch, hydroxypropyl methylcellulose (HPMC) | 1-20 | 2-12 |
| | Disintegrant | Croscarmellose sodium, crospovidone, starch, sodium starch glycollate | 1-20 | 2-12 |
| | Glidant | Talc, colloidal silicon dioxide | 0.1 - 10 | 0.5 - 7.5 |
| | Lubricant | Magnesium stearate, stearic acid, | 0.1 - 10 | 0.5 - 2.5 |
| Coating | Polymer | Hydroxypropyl methylcellulose (HPMC), polyvinyl alcohol (PVA), ethyl cellulose (EC), methyl cellulose (MC), hydroxypropyl cellulose (HPC), acrylate polymers (e.g., Eudragits) | 25 - 55 | 35 - 45 |
| | Plasticizer | Polyethylene glycol (PEG), glycerin, castor oil, propyl gallate (PG), surfactants | 1 - 50 | 20 - 30 |
| | Opacifier | Titanium dioxide, pigment, aluminum lakes, iron oxides, natural colors | 0 - 40 | 10 - 30 |
| | Glidant | Talc, lecithin | 0-15 | 5-15 |

| | | | | |
|---|---|---|---|---|
| * of levels that it may be used (% w/w of total coating material, core tablet weight, or total tablet weight) | | | | |

**Table 8**

| **Proposed Composition of an Immediate Release Tablet Formulation with Water Soluble Antioxidant(s) in the Core of the Tablet** | | | | |
|---|---|---|---|---|
| **Location in tablet** | **Functional class** | **Examples of components** | **Range*** | **Preferred Range*** |
| Core | Active pharmaceutical ingredient | Saxagliptin, sitagliptin, metformin | 1-98 | 5-65 |
| | Filler | Lactose monohydrate, microcrystalline cellulose | 5 - 95 | 20 - 80 |
| | Binder | Hydroxypropyl cellulose (HPC), polyvinyl pyrrolidone (PVP), starch, hydroxypropyl methylcellulose (HPMC) | 1-20 | 2-12 |
| | Disintegrant | Croscarmellose sodium, crospovidone, starch, sodium starch glycollate | 1-20 | 2-12 |
| | Antioxidant | Ascorbic acid, propyl gallate, sodium sulfite, sodium metabisulfite, sodium bisulfite, thioglycerol, thioglycollic acid | 0.001 - 1.0 | 0.01 - 0.5 |
| | Glidant | Talc, colloidal silicon dioxide | 0.1 - 10 | 0.5 - 7.5 |
| | Lubricant | Magnesium stearate, stearic acid, | 0.1 - 10 | 0.5 - 2.5 |
| Coating | Polymer | Hydroxypropyl methylcellulose (HPMC), polyvinyl alcohol (PVA), ethyl cellulose (EC), methyl cellulose (MC), hydroxypropyl cellulose (HPC), acrylate polymers (e.g., Eudragits) | 25 - 55 | 35 - 45 |
| | Plasticizer | Polyethylene glycol (PEG), glycerin, castor oil, propyl gallate (PG), surfactants | 1 - 50 | 20 - 30 |
| | Opacifier | Titanium dioxide, pigment, aluminum lakes, iron oxides, natural colors | 0 - 40 | 10 - 30 |
| | Glidant | Talc, lecithin | 0-15 | 5-15 |

| | | | | |
|---|---|---|---|---|
| * of levels that it may be used (% w/w of total coating material, core tablet weight, or total tablet weight) | | | | |

**Table 9**

| **Proposed Composition of an Immediate Release Tablet Formulation with Water Soluble Antioxidant(s) in the Coating of the Tablet** | | | | |
|---|---|---|---|---|
| **Location in tablet** | **Functional class** | **Examples of components** | **Range*** | **Preferred Range*** |
| Core | Active pharmaceutical ingredient | Saxagliptin, sitagliptin, metformin | 1-98 | 5-65 |
| | Filler | Lactose monohydrate, microcrystalline cellulose | 5 - 95 | 20 - 80 |
| | Binder | Hydroxypropyl cellulose (HPC), polyvinyl pyrrolidone (PVP), starch, hydroxypropyl methylcellulose (HPMC) | 1-20 | 2-12 |
| | Disintegrant | Croscarmellose sodium, crospovidone, starch, sodium starch glycollate | 1-20 | 2-12 |
| | Glidant | Talc, colloidal silicon dioxide | 0.1 - 10 | 0.5 - 7.5 |
| | Lubricant | Magnesium stearate, stearic acid, | 0.1 - 10 | 0.5 - 2.5 |
| Coating | Polymer | Hydroxypropyl methylcellulose (HPMC), polyvinyl alcohol (PVA), ethyl cellulose (EC), methyl cellulose (MC), hydroxypropyl cellulose (HPC), acrylate polymers (e.g., Eudragits) | 25 - 55 | 35 - 45 |
| | Plasticizer | Polyethylene glycol (PEG), glycerin, castor oil, propyl gallate (PG), surfactants | 1 - 50 | 20 - 30 |
| | Opacifier | Titanium dioxide, pigment, aluminum lakes, iron oxides, natural colors | 0 - 40 | 10 - 30 |
| | Antioxidant | Ascorbic acid, propyl gallate, sodium sulfite, sodium metabisulfite, sodium bisulfite, thioglycerol, thioglycollic acid | 0.001 - 1.0 | 0.01 - 0.5 |
| | Glidant | Talc, lecithin | 0-15 | 5-15 |

| | | | | |
|---|---|---|---|---|
| * of levels that it may be used (% w/w of total coating material, core tablet weight, or total tablet weight) | | | | |

**Table 10**

| **Proposed Composition of an Immediate Release Tablet Formulation with Water Soluble Antioxidant(s) in both the Core and the Coating of the Tablet** | | | | |
|---|---|---|---|---|
| **Location in tablet** | **Functional class** | **Examples of components** | **Range*** | **Preferred Range*** |
| Core | Active pharmaceutical ingredient | Saxagliptin, sitagliptin, metformin | 1-98 | 5-65 |
| | Filler | Lactose monohydrate, microcrystalline cellulose | 5 - 95 | 20 - 80 |
| | Binder | Hydroxypropyl cellulose (HPC), polyvinyl pyrrolidone (PVP), starch, hydroxypropyl methylcellulose (HPMC) | 1-20 | 2-12 |
| | Disintegrant | Croscarmellose sodium, crospovidone, starch, sodium starch glycollate | 1-20 | 2-12 |
| | Antioxidant | Ascorbic acid, propyl gallate, sodium sulfite, sodium metabisulfite, sodium bisulfite, thioglycerol, thioglycollic acid | 0.001 - 1.0 | 0.01 - 0.5 |
| | Glidant | Talc, colloidal silicon dioxide | 0.1 - 10 | 0.5 - 7.5 |
| | Lubricant | Magnesium stearate, stearic acid, | 0.1 - 10 | 0.5 - 2.5 |
| Coating | Polymer | Hydroxypropyl methylcellulose (HPMC), polyvinyl alcohol (PVA), ethyl cellulose (EC), methyl cellulose (MC), hydroxypropyl cellulose (HPC), acrylate polymers (e.g., Eudragits) | 25 - 55 | 35 - 45 |
| | Plasticizer | Polyethylene glycol (PEG), glycerin, castor oil, propyl gallate (PG), surfactants | 1 - 50 | 20 - 30 |
| | Opacifier | Titanium dioxide, pigment, aluminum lakes, iron oxides, natural colors | 0 - 40 | 10 - 30 |
| | Antioxidant | Ascorbic acid, propyl gallate, sodium sulfite, sodium metabisulfite, sodium bisulfite, thioglycerol, thioglycollic acid | 0.001 - 1.0 | 0.01 - 0.5 |
| | Glidant | Talc, lecithin | 0-15 | 5-15 |

| | | | | |
|---|---|---|---|---|
| * of levels that it may be used (% w/w of total coating material, core tablet weight, or total tablet weight) | | | | |

**Table 11**

| **Concentration Ranges of Antioxidants that may be used in Drug Product Formulations** | | |
|---|---|---|
| **Antioxidant** | **Ranges (% w/w of total coating material, core tablet weight, or total tablet weight)** | **Preferred Ranges (% w/w of total coating material, core tablet weight, or total tablet weight)** |
| Ascorbic acid | 0.01 - 1.0 | 0.1 - 0.5 |
| Propyl gallate | 0.001 - 1.0 | 0.01 - 0.5 |
| Sodium sulfite | 0.01 - 1.0 | 0.1 - 0.5 |
| Sodium metabisulfite | 0.01 - 1.0 | 0.1 - 0.5 |
| Sodium bisulfite | 0.01 - 1.0 | 0.1 - 0.5 |
| Thioglycerol | 0.01 - 0.5 | 0.02 - 0.25 |
| Thioglycollic acid | 0.01 - 0.5 | 0.02 - 0.25 |

### Example 7

### Saxagliptin Tablet

A saxagliptin tablet is prepared by coating a placebo core tablet with polyvinyl alcohol (PVA)-based Opadry coating material in a three layer coating process to embed saxagliptin in the coating material. A formulation composition of the saxagliptin tablet according to this invention is listed in Table 12. This composition reflects a saxagliptin dose of 2.5 mgs. Saxagliptin tablets of different doses can be prepared by modifying the API loading and composition of the excipients and total tablet weight.

**Table 12**

| **Composition of Saxagliptin Tablet (2.5 mg)** | | |
|---|---|---|
| **Material** | **Function** | **Quantity in Mg/tablet** |
| **Core tablet composition:** | | |
| Lactose monohydrate | Filler | 99 |
| Microcrystalline cellulose | Filler | 90 |
| Croscarmellose sodium | Disintegrant | 10 |
| Magnesium Stearate | Lubricant | 1 |

| **First Layer Composition:** | | |
|---|---|---|
| Opadry® II White | Coating Material | 21 |

| **Second Layer Composition:** | | |
|---|---|---|
| Saxagliptin | API | 2.5 |
| Opadry® II White | Coating Material | 20 |
| Propyl gallate | Antioxidant | 0.5 |

| Third Layer Composition: | | |
|---|---|---|
| Opadry II Colored | Coating Material | 17 |
| Hydrochloric Acid Solution, IN | For pH adjustment | q.s. |
| Purified Water | Vehicle for coating suspension | q.s. |
| Sodium Hydroxide Solution, IN | For pH adjustment | q.s. |
| Opacode | Printing Ink | 0.03 |

### Example 8

### Saxagliptin/Metformin Immediate Release Fixed Dose Tablet

A saxagliptin/metformin hydrochloride immediate release (Saxa/Met IR) tablet is prepared by coating a Met IR core tablet with PVA-based Opadry coating material in a three layer coating process to embed saxagliptin in the coating material. A formulation composition of the Saxa/Met IR tablet is listed in Table 13. This composition reflects saxagliptin dose of 2.5 mg and metformin hydrochloride dose of 500 mg. Other fixed dose combination products can be prepared by modifying the API loading and composition of the excipients in the core or the coating of the tablet and total tablet weight.

**Table 13**

| **Composition of Saxa/Met IR Tablet** | | |
|---|---|---|
| **Material** | **Function** | **Quantity in Mg/tablet** |
| **Core tablet composition:** | | |
| Metformin Hydrochloride | API | 500 |
| Povidone | Binder | 20 |
| Purified Water ca. | Wetting agent | 5 |
| Magnesium Stearate | Lubricant | 1.75 |

| **First Layer Composition:** | | |
|---|---|---|
| Opadry II White | Coating Material | 21 |

| **Second Layer Composition:** | | |
|---|---|---|
| Saxagliptin | API | 2.5 |
| Opadry® II White | Coating Material | 20 |
| Propyl gallate | Antioxidant | 0.5 |

| Third Layer Composition: | | |
|---|---|---|
| Opadry II Colored | Coating Material | 17 |
| Hydrochloric Acid Solution, IN | For pH adjustment | q.s. |
| Purified Water | Vehicle for coating suspension | q.s. |
| Sodium Hydroxide Solution, IN | For pH adjustment | q.s. |
| Opacode | Printing Ink | 0.03 |

### Example 9

### Saxagliptin/Metformin Extended Release Fixed Dose Tablet

A saxagliptin/metformin hydrochloride extended release (Saxa/Met XR) tablet is prepared by coating a Met XR core tablet with PVA-based Opadry coating material in a three layer coating process to embed saxagliptin in the coating material. A formulation composition of the Saxa/Met XR tablet is listed in Table 14. This composition reflects saxagliptin dose of 2.5 mg and metformin hydrochloride dose of 500 mg. Other fixed dose combination products can be prepared by modifying the API loading and composition of the excipients in the core or the coating of the tablet and total tablet weight.

**Table 14**

| **Composition of Saxa/Met XR Tablet** | | |
|---|---|---|
| **Material** | **Function** | **Quantity in Mg/tablet** |
| **Core tablet composition:** | | |
| Metformin Hydrochloride | API | 500 |
| Sodium carboxymethylcellulose | Disintegrant | 50 |
| Hydroxypropyl methylcellulose | Release modifier | 370 |
| Microcrystalline cellulose | Filler | 100 |
| Purified Water ca. | Wetting agent | 5 |
| Magnesium Stearate | Lubricant | 3.5 |

| **First Layer Composition:** | | |
|---|---|---|
| Opadry II White | Coating Material | 21 |

| **Second Layer Composition:** | | |
|---|---|---|
| Saxagliptin | API | 2.5 |
| Opadry® II White | Coating Material | 20 |
| Propyl gallate | Antioxidant | 0.5 |

| **Third Layer Composition:** | | |
|---|---|---|
| Opadry II Colored | Coating Material | 17 |
| Hydrochloric Acid Solution, IN | For pH adjustment | q.s. |
| Purified Water | Vehicle for coating suspension | q.s. |
| Sodium Hydroxide Solution, IN | For pH adjustment | q.s. |
| Opacode | Printing Ink | 0.03 |

### Reference Example 10

### Liquid and Semi-solid Formulations

The formulations of the present disclosure are also applicable to liquid formulations, which may be intended for oral, external, or parenteral use. These formulations can include solutions, suspensions, emulsions, ointments, gels, suppositories, self-emulsifying systems, self-microemulsifying systems, and other semi-solid dosage forms. The liquid formulations of this disclosure can be used for drugs that react with formic acid or a formylating species, such as formic acid esters or anhydrides. Water soluble antioxidants may be added to formulations that contain residual levels of formic acid or a different formylating species (in the range of 5 ppm or higher). The formulations may contain a formylating species initially and/or they may be formed on storage at high temperature (such as 40 °C to 60 °C) and/or humidity (such as 75% RH to 95% RH) conditions over a period of 2 days to 3 months.

## Claims

1. A coated tablet comprising:
(a) a tablet core wherein the tablet core comprises
(i) optionally at least one antidiabetic agent or a pharmaceutically acceptable salt thereof, wherein the antidiabetic agent is other than saxagliptin;
(b) a first layer that coats the tablet core, wherein the first layer comprises
(i) a coating material; and
(ii) optionally at least one water soluble antioxidant;
(c) a second layer that coats the first layer wherein the second layer comprises
(i) a coating material comprising at least 25 wt% of a combination of poly(ethylene glycol) and poly(vinyl alcohol);
(ii) at least one water soluble antioxidant selected from ascorbic acid, propyl gallate, or a combination thereof; and
(iii) saxagliptin or a pharmaceutically acceptable salt thereof; and
(d) a third layer that coats the second layer wherein the third layer comprises
(i) a coating material; and
(ii) optionally at least one water soluble antioxidant.

2. The coated tablet according to claim 1 wherein the saxagliptin is provided as saxagliptin hydrochloride.

3. The coated tablet according to claim 1 or claim 2, wherein the water soluble antioxidant is propyl gallate.

4. The coated tablet according to any of claims 1-3, wherein the coating material of the second coating layer comprises at least 50 wt% of a combination of poly(vinyl alcohol) and poly(ethylene glycol).

5. The coated tablet according to any of claims 1-4, wherein the tablet core comprises two fillers, a disintegrant, and a lubricant, and wherein the coating material of the first layer, the second layer, and the third layer is a coating material comprising polyvinyl alcohol, titanium dioxide, polyethylene glycol and talc.

6. The coated tablet according to claim 1 wherein:
(a) the tablet core comprises
(i) 99 mgs of lactose monohydrate;
(ii) 90 mgs of microcrystalline cellulose;
(iii) 10 mgs of croscarmellose sodium; and
(iv) 1 mg of magnesium stearate;
(b) the first layer comprises
(i) 21 mgs of a coating material comprised of polyvinyl alcohol, titanium dioxide, polyethylene glycol and talc;
(c) the second layer comprises
(i) 20 mgs of a coating material comprised of polyvinyl alcohol, titanium dioxide, polyethylene glycol and talc;
(ii) 0.5 mgs propyl gallate; and
(iii) 2.5 mgs saxagliptin; and
(d) the third layer comprises
(i) 17 mgs of a colored coating material comprising polyvinyl alcohol, titanium dioxide, polyethylene glycol and talc.

7. The coated tablet according to any of claims 1-5, wherein the tablet core comprises a binder, a wetting agent, a lubricant, and at least one antidiabetic or a pharmaceutically acceptable salt thereof, wherein the antidiabetic is glyburide, metformin, rosiglitazone, pioglitazone, sitagliptin, vildagliptin, dapagliflozin, dapagliflozin (S) propylene glycol hydrate, or a combination thereof.

8. The coated tablet according to any of claims 1-5, wherein the tablet core comprises a binder, a wetting agent, a lubricant, and an antidiabetic, wherein the antidiabetic is metformin, dapagliflozin, dapagliflozin (S) propylene glycol hydrate, or a combination thereof.

9. The coated tablet according to claim 8, wherein the water soluble antioxidant is ascorbic acid or propyl gallate, and wherein the coating material is a material comprising polyvinyl alcohol, titanium dioxide, polyethylene glycol and talc.

10. The coated tablet according to claim 1 wherein:
(a) the tablet core comprises dapagliflozin or dapagliflozin (S) propylene glycol hydrate, a binder, a wetting agent, and a lubricant;
(b) the first layer comprises a coating material comprised of polyvinyl alcohol, titanium dioxide, polyethylene glycol and talc;
(c) the second layer comprises a coating material comprised of polyvinyl alcohol, titanium dioxide, polyethylene glycol and talc; ascorbic acid or propyl gallate; and saxagliptin; and
(d) the third layer comprises a colored coating material comprising polyvinyl alcohol, titanium dioxide, polyethylene glycol and talc.

11. The coated tablet according to claim 1 wherein:
(a) the tablet core comprises
(1) dapagliflozin or dapagliflozin (S) propylene glycol hydrate, povidone, purified water, and magnesium stearate;
(2) metformin hydrochloride, a binder, a wetting agent and a lubricant;
(3) metformin hydrochloride, povidone, purified water and magnesium stearate;
(4) dapagliflozin or dapagliflozin (S) propylene glycol hydrate, sodium carboxymethylcellulose, hydroxypropyl methylcellulose, microcrystalline cellulose, purified water and magnesium stearate;
(5) metformin hydrochloride, a disintegrant, a release modifier, a filler, a wetting agent and a lubricant;
(6) metformin hydrochloride, sodium carboxymethylcellulose, hydroxypropyl methylcellulose, microcrystalline cellulose, purified water and magnesium stearate; or
(7) dapagliflozin or dapagliflozin (S) propylene glycol hydrate, a disintegrant, a release modifier, a filler, a wetting agent and a lubricant;
(b) the first layer comprises a coating material comprised of polyvinyl alcohol, titanium dioxide, polyethylene glycol and talc;
(c) the second layer comprises a coating material comprised of polyvinyl alcohol, titanium dioxide, polyethylene glycol and talc; ascorbic acid or propyl gallate; and saxagliptin; and
(d) the third layer comprises a colored coating material comprising polyvinyl alcohol, titanium dioxide, polyethylene glycol and talc.

12. The coated tablet according to claim 1 wherein:
(a) the tablet core comprises
(i) 500 mgs of metformin hydrochloride;
(ii) 20 mgs of Povidone;
(iii) 5 mgs of purified water; and
(iv) 1.75 mgs of magnesium stearate;
(b) the first layer comprises
(i) 21 mgs of a coating material comprised of polyvinyl alcohol, titanium dioxide, polyethylene glycol and talc;
(c) the second layer comprises
(i) 20 mgs of a coating material comprised of polyvinyl alcohol, titanium dioxide, polyethylene glycol and talc;
(ii) 0.5 mgs propyl gallate; and
(iii) 2.5 mgs saxagliptin; and
(d) the third layer comprises
(i) 17 mgs of a colored coating material comprising polyvinyl alcohol, titanium dioxide, polyethylene glycol and talc.

13. The coated tablet according to any of claims 1-5, wherein the tablet core comprises a disintegrant, a release modifier, a filler, a wetting agent, a lubricant, and at least one antidiabetic or a pharmaceutically acceptable salt thereof, wherein the antidiabetic is glyburide, metformin, rosiglitazone, pioglitazone, sitagliptin, vildagliptin, dapagliflozin, dapagliflozin (S) propylene glycol hydrate, or a combination thereof.

14. The coated tablet according to any of claims 1-5, wherein the tablet core comprises a disintegrant, a release modifier, a filler, a wetting agent, a lubricant, and an antidiabetic, wherein the antidiabetic is metformin, dapagliflozin, dapagliflozin (S) propylene glycol hydrate, or a combination thereof.

15. The coated tablet according to claim 14, wherein the water soluble antioxidant is ascorbic acid or propyl gallate, and wherein the coating material is a material comprising polyvinyl alcohol, titanium dioxide, polyethylene glycol and talc.

16. The coated tablet according to claim 1 wherein:
(a) the tablet core comprises
(i) 500 mgs of metformin hydrochloride;
(ii) 50 mgs of sodium carboxymethylcellulose;
(iii) 370 mgs of hydroxypropyl methylcellulose;
(iv) 100 mgs of microcrystalline cellulose;
(v) 5 mgs of purified water; and
(vi) 3.5 mgs of magnesium stearate;
(b) the first layer comprises
(i) 21 mgs of a coating material comprised of polyvinyl alcohol, titanium dioxide, polyethylene glycol and talc;
(c) the second layer comprises
(i) 20 mgs of a coating material comprised of polyvinyl alcohol, titanium dioxide, polyethylene glycol and talc;
(ii) 0.5 mgs propyl gallate; and
(iii) 2.5 mgs saxagliptin; and
(d) the third layer comprises
(i) 17 mgs of a colored coating material comprising polyvinyl alcohol, titanium dioxide, polyethylene glycol and talc.

## Patentansprüche

1. Beschichtete Tablette, umfassend:
(a) einen Tablettenkern, wobei der Tablettenkern Folgendes umfasst
(i) wahlweise mindestens ein Antidiabetikum oder ein pharmazeutisch verträgliches Salz davon, wobei das Antidiabetikum von Saxagliptin verschieden ist;
(b) eine erste Schicht, die den Tablettenkern beschichtet, wobei die erste Schicht Folgendes umfasst
(i) ein Beschichtungsmaterial; und
(ii) wahlweise mindestens ein wasserlösliches Antioxidationsmittel;
(c) eine zweite Schicht, die die erste Schicht beschichtet, wobei die zweite Schicht Folgendes umfasst
(i) ein Beschichtungsmaterial, das zu mindestens 25 Gew.-% eine Kombination von Poly(ethylenglycol) und Poly(vinylalkohol) umfasst
(ii) mindestens ein wasserlösliches Antioxidationsmittel, ausgewählt aus Ascorbinsäure, Propylgallat oder eine Kombination davon; und
(iii) Saxagliptin oder ein pharmazeutisch verträgliches Salz davon; und
(d) eine dritte Schicht, die die zweite Schicht beschichtet, wobei die dritte Schicht Folgendes umfasst
(i) ein Beschichtungsmaterial; und
(ii) wahlweise mindestens ein wasserlösliches Antioxidationsmittel.

2. Beschichtete Tablette nach Anspruch 1, wobei das Saxagliptin als Saxagliptinhydrochlorid bereitgestellt ist.

3. Beschichtete Tablette nach Anspruch 1 oder Anspruch 2, wobei das wasserlösliche Antioxidationsmittel Propylgallat ist.

4. Beschichtete Tablette nach einem der Ansprüche 1-3, wobei das Beschichtungsmaterial der zweiten Beschichtungsschicht zu mindestens 50 Gew.-% eine Kombination von Poly(vinylalkohol) und Poly(ethylenglycol) umfasst.

5. Beschichtete Tablette nach einem der Ansprüche 1-4, wobei der Tablettenkern zwei Füllstoffe, ein Zersetzungsmittel und ein Schmiermittel umfasst und wobei das Beschichtungsmaterial der ersten Schicht, der zweiten Schicht und der dritten Schicht ein Beschichtungsmaterial ist, das Polyvinylalkohol, Titandioxid, Polyethylenglycol und Talk umfasst.

6. Beschichtete Tablette nach Anspruch 1, wobei:
(a) der Tablettenkern Folgendes umfasst
(i) 99 mg Lactosemonohydrat;
(ii) 90 mg mikrokristalline Cellulose;
(iii) 10 mg Croscarmellose-Natrium; und
(iv) 1 mg Magnesiumstearat;
(b) die erste Schicht Folgendes umfasst
(i) 21 mg eines Beschichtungsmaterials, bestehend aus Polyvinylalkohol, Titandioxid, Polyethylenglycol und Talk;
(c) die zweite Schicht Folgendes umfasst
(i) 20 mg eines Beschichtungsmaterials, bestehend aus Polyvinylalkohol, Titandioxid, Polyethylenglycol und Talk;
(ii) 0,5 mg Propylgallat; und
(iii) 2,5 mg Saxagliptin; und
(d) die dritte Schicht Folgendes umfasst
(i) 17 mg eines farbigen Beschichtungsmaterials, umfassend Polyvinylalkohol, Titandioxid, Polyethylenglycol und Talk.

7. Beschichtete Tablette nach einem der Ansprüche 1-5, wobei der Tablettenkern ein Bindemittel, ein Benetzungsmittel, ein Schmiermittel und mindestens ein Antidiabetikum oder ein pharmazeutisch verträgliches Salz davon umfasst, wobei das Antidiabetikum Glyburid, Metformin, Rosiglitazon, Pioglitazon, Sitagliptin, Vildagliptin, Dapagliflozin, Dapagliflozin-(S)-Propylenglycolhydrat oder eine Kombination davon ist.

8. Beschichtete Tablette nach einem der Ansprüche 1-5, wobei der Tablettenkern ein Bindemittel, ein Benetzungsmittel, ein Schmiermittel und ein Antidiabetikum umfasst, wobei das Antidiabetikum Metformin, Dapagliflozin, Dapagliflozin-(S)-Propylenglycolhydrat oder eine Kombination davon ist.

9. Beschichtete Tablette nach Anspruch 8, wobei das wasserlösliche Antioxidationsmittel Ascorbinsäure oder Propylgallat ist und wobei das Beschichtungsmaterial ein Material ist, das Polyvinylalkohol, Titandioxid, Polyethylenglycol und Talk umfasst.

10. Beschichtete Tablette nach Anspruch 1, wobei:
(a) der Tablettenkern Dapagliflozin oder Dapagliflozin-(S)-Propylenglycolhydrat, ein Bindemittel, ein Benetzungsmittel und ein Schmiermittel umfasst;
(b) die erste Schicht ein Beschichtungsmaterial, bestehend aus Polyvinylalkohol, Titandioxid, Polyethylenglycol und Talk, umfasst;
(c) die zweite Schicht ein Beschichtungsmaterial, bestehend aus Polyvinylalkohol, Titandioxid, Polyethylenglycol und Talk; Ascorbinsäure oder Propylgallat; und Saxagliptin umfasst; und
(d) die dritte Schicht ein farbiges Beschichtungsmaterial, umfassend Polyvinylalkohol, Titandioxid, Polyethylenglycol und Talk, umfasst.

11. Beschichtete Tablette nach Anspruch 1, wobei:
(a) der Tablettenkern Folgendes umfasst
(1) Dapagliflozin oder Dapagliflozin-(S)-Propylenglycolhydrat, Povidon, gereinigtes Wasser und Magnesiumstearat;
(2) Metforminhydrochlorid, ein Bindemittel, ein Benetzungsmittel und ein Schmiermittel;
(3) Metforminhydrochlorid, Povidon, gereinigtes Wasser und Magnesiumstearat;
(4) Dapagliflozin oder Dapagliflozin-(S)-Propylenglycolhydrat, Natriumcarboxymethylcellulose, Hydroxypropylmethylcellulose, mikrokristalline Cellulose, gereinigtes Wasser und Magnesiumstearat;
(5) Metforminhydrochlorid, ein Zersetzungsmittel, ein Trennmodifizierungsmittel, einen Füllstoff, ein Benetzungsmittel und ein Schmiermittel;
(6) Metforminhydrochlorid, Natriumcarboxymethylcellulose, Hydroxypropylmethylcellulose, mikrokristalline Cellulose, gereinigtes Wasser und Magnesiumstearat; oder
(7) Dapagliflozin oder Dapagliflozin-(S)-Propylenglycolhydrat, ein Zersetzungsmittel, ein Trennmodifizierungsmittel, einen Füllstoff, ein Benetzungsmittel und ein Schmiermittel;
(b) die erste Schicht ein Beschichtungsmaterial, bestehend aus Polyvinylalkohol, Titandioxid, Polyethylenglycol und Talk, umfasst;
(c) die zweite Schicht ein Beschichtungsmaterial, bestehend aus Polyvinylalkohol, Titandioxid, Polyethylenglycol und Talk; Ascorbinsäure oder Propylgallat; und Saxagliptin umfasst; und
(d) die dritte Schicht ein farbiges Beschichtungsmaterial, umfassend Polyvinylalkohol, Titandioxid, Polyethylenglycol und Talk, umfasst.

12. Beschichtete Tablette nach Anspruch 1, wobei:
(a) der Tablettenkern Folgendes umfasst
(i) 500 mg Metforminhydrochlorid;
(ii) 20 mg Povidon;
(iii) 5 mg gereinigtes Wasser; und
(iv) 1,75 mg Magnesiumstearat;
(b) die erste Schicht Folgendes umfasst
(i) 21 mg eines Beschichtungsmaterials, bestehend aus Polyvinylalkohol, Titandioxid, Polyethylenglycol und Talk;
(c) die zweite Schicht Folgendes umfasst
(i) 20 mg eines Beschichtungsmaterials, bestehend aus Polyvinylalkohol, Titandioxid, Polyethylenglycol und Talk;
(ii) 0,5 mg Propylgallat; und
(iii) 2,5 mg Saxagliptin; und
(d) die dritte Schicht Folgendes umfasst
(i) 17 mg eines farbigen Beschichtungsmaterials, umfassend Polyvinylalkohol, Titandioxid, Polyethylenglycol und Talk.

13. Beschichtete Tablette nach einem der Ansprüche 1-5, wobei der Tablettenkern ein Zersetzungsmittel, ein Trennmodifizierungsmittel, einen Füllstoff, ein Benetzungsmittel, ein Schmiermittel und mindestens ein Antidiabetikum oder ein pharmazeutisch verträgliches Salz davon umfasst, wobei das Antidiabetikum Glyburid, Metformin, Rosiglitazon, Pioglitazon, Sitagliptin, Vildagliptin, Dapagliflozin, Dapagliflozin-(S)-Propylenglycolhydrat oder eine Kombination davon ist.

14. Beschichtete Tablette nach einem der Ansprüche 1-5, wobei der Tablettenkern ein Zersetzungsmittel, ein Trennmodifizierungsmittel, einen Füllstoff, ein Benetzungsmittel, ein Schmiermittel und ein Antidiabetikum umfasst, wobei das Antidiabetikum Metformin, Dapagliflozin, Dapagliflozin-(S)-Propylenglycolhydrat oder eine Kombination davon ist.

15. Beschichtete Tablette nach Anspruch 14, wobei das wasserlösliche Antioxidationsmittel Ascorbinsäure oder Propylgallat ist und wobei das Beschichtungsmaterial ein Material ist, das Polyvinylalkohol, Titandioxid, Polyethylenglycol und Talk umfasst.

16. Beschichtete Tablette nach Anspruch 1, wobei:
(a) der Tablettenkern Folgendes umfasst
(i) 500 mg Metforminhydrochlorid;
(ii) 50 mg Natriumcarboxymethylcellulose;
(iii) 370 mg Hydroxypropylmethylcellulose;
(iv) 100 mg mikrokristalline Cellulose;
(v) 5 mg gereinigtes Wasser; und
(vi) 3,5 mg Magnesiumstearat;
(b) die erste Schicht Folgendes umfasst
(i) 21 mg eines Beschichtungsmaterials, bestehend aus Polyvinylalkohol, Titandioxid, Polyethylenglycol und Talk;
(c) die zweite Schicht Folgendes umfasst
(i) 20 mg eines Beschichtungsmaterials, bestehend aus Polyvinylalkohol, Titandioxid, Polyethylenglycol und Talk;
(ii) 0,5 mg Propylgallat; und
(iii) 2,5 mg Saxagliptin; und
(d) die dritte Schicht Folgendes umfasst
(i) 17 mg eines farbigen Beschichtungsmaterials, umfassend Polyvinylalkohol, Titandioxid, Polyethylenglycol und Talk.

## Revendications

1. Comprimé enrobé comprenant :
(a) un noyau de comprimé dans lequel le noyau de comprimé comprend
(i) facultativement au moins un agent antidiabétique ou un sel pharmaceutiquement acceptable de celui-ci, dans lequel l'agent antidiabétique est autre que la saxagliptine ;
(b) une première couche qui enrobe le noyau de comprimé, la première couche comprenant
(i) un matériau d'enrobage ; et
(ii) facultativement au moins un antioxydant soluble dans l'eau ;
(c) une deuxième couche qui enrobe la première couche, la deuxième couche comprenant
(i) un matériau d'enrobage comprenant au moins 25 % en poids d'une combinaison de poly(éthylène glycol) et de poly(alcool vinylique) ;
(ii) au moins un antioxydant soluble dans l'eau choisi parmi l'acide ascorbique, le gallate de propyle ou une combinaison de ceux-ci ; et
(iii) de la saxagliptine ou un sel pharmaceutiquement acceptable de celle-ci ; et
(d) une troisième couche qui enrobe la deuxième couche, la troisième couche comprenant
(i) un matériau d'enrobage ; et
(ii) facultativement au moins un antioxydant soluble dans l'eau.

2. Comprimé enrobé selon la revendication 1, dans lequel la saxagliptine est fournie sous la forme de chlorhydrate de saxagliptine.

3. Comprimé enrobé selon la revendication 1 ou la revendication 2, dans lequel l'antioxydant soluble dans l'eau est du gallate de propyle.

4. Comprimé enrobé selon l'une quelconque des revendications 1 à 3, dans lequel le matériau d'enrobage de la seconde couche d'enrobage comprend au moins 50 % en poids d'une combinaison de poly(alcool vinylique) et de poly(éthylène glycol).

5. Comprimé enrobé selon l'une quelconque des revendications 1 à 4, dans lequel le noyau de comprimé comprend deux agents de remplissage, un délitant et un lubrifiant, et dans lequel le matériau d'enrobage de la première couche, de la deuxième couche et de la troisième couche est un matériau d'enrobage comprenant de l'alcool polyvinylique, du dioxyde de titane, du polyéthylène glycol et du talc.

6. Comprimé enrobé selon la revendication 1, dans lequel :
(a) le noyau de comprimé comprend
(i) 99 mg de lactose monohydraté ;
(ii) 90 mg de cellulose microcristalline ;
(iii) 10 mg de croscarmellose sodique ; et
(iv) 1 mg de stéarate de magnésium ;
(b) la première couche comprend
(i) 21 mg d'un matériau d'enrobage composé d'alcool polyvinylique, de dioxyde de titane, de polyéthylène glycol et de talc ;
(c) la deuxième couche comprend
(i) 20 mg d'un matériau d'enrobage composé d'alcool polyvinylique, de dioxyde de titane, de polyéthylène glycol et de talc ;
(ii) 0,5 mg de gallate de propyle ; et
(iii) 2,5 mg de saxagliptine ; et
(d) la troisième couche comprend
(i) 17 mg d'un matériau d'enrobage coloré comprenant de l'alcool polyvinylique, du dioxyde de titane, du polyéthylène glycol et du talc.

7. Comprimé enrobé selon l'une quelconque des revendications 1 à 5, dans lequel le noyau de comprimé comprend un liant, un agent mouillant, un lubrifiant, et au moins un agent antidiabétique ou un sel pharmaceutiquement acceptable de celui-ci, dans lequel l'agent antidiabétique est du glyburide, de la metformine, de la rosiglitazone, de la pioglitazone, de la sitagliptine, de la vildagliptine, de la dapagliflozine, du dapagliflozine (S) propylène glycol hydraté, ou une combinaison de ceux-ci.

8. Comprimé enrobé selon l'une quelconque des revendications 1 à 5, dans lequel le noyau de comprimé comprend un liant, un agent mouillant, un lubrifiant et un agent antidiabétique, dans lequel l'agent antidiabétique est de la metformine, de la dapagliflozine, du dapagliflozine (S) propylène glycol hydraté, ou une combinaison de ceux-ci.

9. Comprimé enrobé selon la revendication 8, dans lequel l'antioxydant soluble dans l'eau est de l'acide ascorbique ou du gallate de propyle, et dans lequel le matériau d'enrobage est un matériau comprenant de l'alcool polyvinylique, du dioxyde de titane, du polyéthylène glycol et du talc.

10. Comprimé enrobé selon la revendication 1, dans lequel :
(a) le noyau de comprimé comprend de la dapagliflozine ou du dapagliflozine (S) propylène glycol hydraté, un liant, un agent mouillant et un lubrifiant ;
(b) la première couche comprend un matériau d'enrobage composé d'alcool polyvinylique, de dioxyde de titane, de polyéthylène glycol et de talc ;
(c) la deuxième couche comprend un matériau d'enrobage composé d'alcool polyvinylique, de dioxyde de titane, de polyéthylène glycol et de talc ; de l'acide ascorbique ou du gallate de propyle ; et de la saxagliptine ; et
(d) la troisième couche comprend un matériau d'enrobage coloré comprenant de l'alcool polyvinylique, du dioxyde de titane, du polyéthylène glycol et du talc.

11. Comprimé enrobé selon la revendication 1, dans lequel :
(a) le noyau de comprimé comprend
(1) de la dapagliflozine ou du dapagliflozine (S) propylène glycol hydraté, de la povidone, de l'eau purifiée et du stéarate de magnésium ;
(2) du chlorhydrate de metformine, un liant, un agent mouillant et un lubrifiant ;
(3) du chlorhydrate de metformine, de la povidone, de l'eau purifiée et du stéarate de magnésium ;
(4) de la dapagliflozine ou du dapagliflozine (S) propylène glycol hydraté, de la carboxyméthylcellulose sodique, de l'hydroxypropylméthylcellulose, de la cellulose microcristalline, de l'eau purifiée et du stéarate de magnésium ;
(5) du chlorhydrate de metformine, un délitant, un modificateur de libération, un agent de remplissage, un agent mouillant et un lubrifiant ;
(6) du chlorhydrate de metformine, de la carboxyméthylcellulose sodique, de l'hydroxypropylméthylcellulose, de la cellulose microcristalline, de l'eau purifiée et du stéarate de magnésium ; ou
(7) de la dapagliflozine ou du dapagliflozine (S) propylène glycol hydraté, un délitant, un modificateur de libération, un agent de remplissage, un agent mouillant et un lubrifiant ;
(b) la première couche comprend un matériau d'enrobage composé d'alcool polyvinylique, de dioxyde de titane, de polyéthylène glycol et de talc ;
(c) la deuxième couche comprend un matériau d'enrobage composé d'alcool polyvinylique, de dioxyde de titane, de polyéthylène glycol et de talc ; de l'acide ascorbique ou du gallate de propyle ; et de la saxagliptine ; et
(d) la troisième couche comprend un matériau d'enrobage coloré comprenant de l'alcool polyvinylique, du dioxyde de titane, du polyéthylène glycol et du talc.

12. Comprimé enrobé selon la revendication 1, dans lequel :
(a) le noyau de comprimé comprend
(i) 500 mg de chlorhydrate de metformine ;
(ii) 20 mg de Povidone ;
(iii) 5 mg d'eau purifiée ; et
(iv) 1,75 mg de stéarate de magnésium ;
(b) la première couche comprend
(i) 21 mg d'un matériau d'enrobage composé d'alcool polyvinylique, de dioxyde de titane, de polyéthylène glycol et de talc ;
(c) la deuxième couche comprend
(i) 20 mg d'un matériau d'enrobage composé d'alcool polyvinylique, de dioxyde de titane, de polyéthylène glycol et de talc ;
(ii) 0,5 mg de gallate de propyle ; et
(iii) 2,5 mg de saxagliptine ; et
(d) la troisième couche comprend
(i) 17 mg d'un matériau d'enrobage coloré comprenant de l'alcool polyvinylique, du dioxyde de titane, du polyéthylène glycol et du talc.

13. Comprimé enrobé selon l'une quelconque des revendications 1 à 5, dans lequel le noyau de comprimé comprend un délitant, un modificateur de libération, un agent de remplissage, un agent mouillant, un lubrifiant, et au moins un agent antidiabétique ou un sel pharmaceutiquement acceptable de celui-ci, dans lequel l'agent antidiabétique est du glyburide, de la metformine, de la rosiglitazone, de la pioglitazone, de la sitagliptine, de la vildagliptine, de la dapagliflozine, du dapagliflozine (S) propylène glycol hydraté, ou une combinaison de ceux-ci.

14. Comprimé enrobé selon l'une quelconque des revendications 1 à 5, dans lequel le noyau de comprimé comprend un délitant, un modificateur de libération, un agent de remplissage, un agent mouillant, un lubrifiant et un agent antidiabétique, dans lequel l'agent antidiabétique est de la metformine, de la dapagliflozine, du dapagliflozine (S) propylène glycol hydraté, ou une combinaison de ceux-ci.

15. Comprimé enrobé selon la revendication 14, dans lequel l'antioxydant soluble dans l'eau est de l'acide ascorbique ou du gallate de propyle, et dans lequel le matériau d'enrobage est un matériau comprenant de l'alcool polyvinylique, du dioxyde de titane, du polyéthylène glycol et du talc.

16. Comprimé enrobé selon la revendication 1, dans lequel :
(a) le noyau de comprimé comprend
(i) 500 mg de chlorhydrate de metformine ;
(ii) 50 mg de carboxyméthylcellulose sodique ;
(iii) 370 mg d'hydroxypropylméthylcellulose ;
(iv) 100 mg de cellulose microcristalline ;
(v) 5 mg d'eau purifiée ; et
(vi) 3,5 mg de stéarate de magnésium ;
(b) la première couche comprend
(i) 21 mg d'un matériau d'enrobage composé d'alcool polyvinylique, de dioxyde de titane, de polyéthylène glycol et de talc ;
(c) la deuxième couche comprend
(i) 20 mg d'un matériau d'enrobage composé d'alcool polyvinylique, de dioxyde de titane, de polyéthylène glycol et de talc ;
(ii) 0,5 mg de gallate de propyle ; et
(iii) 2,5 mg de saxagliptine ; et
(d) la troisième couche comprend
(i) 17 mg d'un matériau d'enrobage coloré comprenant de l'alcool polyvinylique, du dioxyde de titane, du polyéthylène glycol et du talc.
